(19) **Europäisches Patentamt / European Patent Office / Office européen des brevets**

(11) **EP 3 642 599 B1**

(12) **EUROPEAN PATENT SPECIFICATION**

(45) Date of publication and mention
of the grant of the patent:
**08.01.2025 Bulletin 2025/02**

(21) Application number: **18752302.2**

(22) Date of filing: **22.06.2018**

(51) International Patent Classification (IPC):
*G01N 21/64* (2006.01)     *A61K 49/00* (2006.01)

(52) Cooperative Patent Classification (CPC):
**G01N 21/6428; A61K 49/0021; A61K 49/0043;
A61K 49/0056; A61K 49/0093;** G01N 2021/6421

(86) International application number:
**PCT/US2018/038973**

(87) International publication number:
**WO 2018/237253 (27.12.2018 Gazette 2018/52)**

(54) **METHOD OF IMAGING IN VIVO TISSUES USING NANOPARTICLES COMPRISING A REFERENCE DYE AND A SENSOR DYE.**

VERFAHREN ZUR ABBILDUNG VON GEWEBEN IN VIVO UNTER VERWENDUNG VON NANOPARTIKELN MIT EINEM REFERENZFARBSTOFF UND EINEM SENSORFARBSTOFF

PROCÉDÉ D'IMAGERIE DE TISSUS IN VIVO À L'AIDE DE NANOPARTICULES COMPRENANT UN COLORANT DE RÉFÉRENCE ET UN COLORANT DE CAPTEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2017 US 201762524441 P
24.11.2017 US 201762590507 P**

(43) Date of publication of application:
**29.04.2020 Bulletin 2020/18**

(73) Proprietors:
• **Memorial Sloan-Kettering Cancer Center
New York, NY 10065 (US)**
• **Cornell University
Ithaca, NY 14850 (US)**

(72) Inventors:
• **BRADBURY, Michelle S.
New York
New York 10065 (US)**
• **WIESNER, Ulrich
Ithaca
New York 14850 (US)**
• **OVERHOLTZER, Michael
New York
New York 10065 (US)**
• **ABU-RUSTUM, Nadeem R.
New York
New York 10065 (US)**
• **MADAJEWSKI, Brian
New York
New York 10065 (US)**

(74) Representative: **Graham Watt & Co LLP
St. Botolph's House
7-9 St. Botolph's Road
Sevenoaks TN13 3AJ (GB)**

(56) References cited:
**WO-A1-2008/044138         WO-A1-2018/213851
US-A1- 2006 173 362         US-A1- 2006 245 971
US-A1- 2016 202 185**

• **E. PHILLIPS ET AL: "Clinical translation of an ultrasmall inorganic optical-PET imaging nanoparticle probe", SCIENCE TRANSLATIONAL MEDICINE, vol. 6, no. 260, 29 October 2014 (2014-10-29), pages 260ra149 - 260ra149, XP055254613, ISSN: 1946-6234, DOI: 10.1126/ scitranslmed.3009524**

- B. YOO ET AL: "Expanding analytical tools for characterizing ultrasmall silica-based nanoparticles", RSC ADVANCES, vol. 7, no. 27, 1 January 2017 (2017-01-01), pages 16861 - 16865, XP055506548, DOI: 10.1039/C7RA01349C
- ISABELLA TAVERNARO ET AL: "Bright fluorescent silica-nanoparticle probes for high-resolution STED and confocal microscopy", BEILSTEIN JOURNAL OF NANOTECHNOLOGY, vol. 8, 21 June 2017 (2017-06-21), pages 1283 - 1296, XP055506649, DOI: 10.3762/bjnano.8.130
- SUNG EUN KIM ET AL: "Ultrasmall nanoparticles induce ferroptosis in nutrient-deprived cancer cells and suppress tumour growth", NATURE NANOTECHNOLOGY, vol. 11, no. 11, 26 September 2016 (2016-09-26), GB, pages 977 - 985, XP055507126, ISSN: 1748-3387, DOI: 10.1038/nnano.2016.164

## Description

### Cross-Reference to Related Applications

This application claims the benefit of U.S. Application Serial No. 62/524,441 filed on June 23, 2017 and U.S. Application Serial No. 62/590,507 filed on November 24, 2017.

### Field of the Invention

This invention relates generally to methods for super-resolution, intracellular imaging, assessment, and/or treatment of tissue before, during, and/or after surgical procedures using nanoparticles less than 10 nanometers in diameter, e.g., photoswitchable nanoparticles, e.g., nanosensors).

### Background

A medical practitioner must often assess the nature of (e.g., cancerous, non-cancerous) and/or viability of a region of remaining tissue before, during, or after tumor excision or other tissue removal surgery.

Existing techniques for assessing viability involve use of Doppler ultrasound and/or CT/PET/SPECT imaging systems. However, these imaging systems are limited to detection of large vessels only, and lack the sensitivity required to distinguish tiny vessels feeding a graft. Moreover, these imaging systems are inconvenient and generally cannot be utilized during surgery.

Burns et al. "Photoluminescent Silica-Based Sensors and Methods of Use," in US Patent No. US 8,084,001 B1 describes particles for pH sensing. However, Burns et al. does not address subcellular applications. The particles used in Burns are not appropriate for *in vivo* use, for example, because the particles would not diffuse sufficiently well between in vivo tissue compartments, they cannot be used to uniformly assess pH or other properties of a particular region or area, and they would not allow renal

Document WO 2018/213851 A1, published on 22 November 2018 with a valid effective filing date of 19 May 2017 is part of the prior art according to Art.54(3) EPC and discloses an imaging method at the sub-cellular level using ultra-small nanoparticles provided with a reference photoluminescent dye and a sensing photoluminescent dye. However there are no counting of individual nanoparticles.

There remains a need for imaging systems and methods with high sensitivity for assessment of remaining tissue during and/or after tissue (e.g., tumor tissue) removed during surgical procedures. There is also a need for apparatus, systems, and methods for *in vivo* monitoring of cell/tissue viability following surgery, and treating such tissue on an as-needed basis.

## Summary

Described herein are systems and methods for intracellular imaging, assessment, and/or treatment of tissue before, during, and/or after surgical procedures using nanoparticles (less than 10 nanometers in diameter, e.g., photoswitchable nanoparticles) and/or a super-resolution microscope system.

The present disclosure describes nanoparticles (e.g., nanosensors and photoswitchable nanoparticles) that are used to monitor and/or track changes in environmental conditions and/or analytes in the cellular microenvironment before, during, and/or after surgical procedures. The nanoparticles detect changes in reactive oxygen species (ROS), pH, pH perturbations, iron levels, calcium, glutathione, and/or amino acids such as leucine, glutamine, arginine, and others in the sub-cellular microenvironment. The systems and methods may provide a map of perfusion, perfusion alterations, and/or oxygen/pH status before, during, and/or after surgery. Assessment of analytes may be qualitative or quantitative.

The present disclosure also describes systems and methods that provide information related to the distribution and/or delivery of photoswitchable nanoparticles at super resolution (e.g., using super-resolution microscopy). Distribution and/or delivery of nanoparticles is determined by counting and/or tracking the number of nanoparticles localized within a subcellular compartment, structure, and/or across the blood-brain barrier.

The ability to count nanoparticles and localize them within or outside of a cellular compartment, structure, and/or within/across biological barriers at super resolution (i) helps to assess unanticipated events (e.g., effects caused by too little or too many nanoparticles localized within the cell and/or cellular compartment), (ii) can be done patient-by-patient at a cellular level, and (iii) can be coupled with proteomics and/or genomics for improved personalized medicine and care.

Therefore, the technology can provide useful information to determine dose limits for drug delivery, and/or can facilitate design of nanoparticle surface chemistry to maximize nanoparticle delivery and/or therapeutic response. Moreover, the technology facilitates analysis of a cell's microenvironment, and can help obtain criteria to stratify patients and produce compositions having an appropriate surface chemistry tailored for an individual patient.

In one aspect, the invention is directed to a method as defined in claim 1.

In certain embodiments, the nanosensor comprises a pathway inhibitor and/or other immune modulator (and, optionally, a targeting agent).

In certain embodiments, each dye comprises an independently-detectable fluorophore. In certain embodiments, each dye emits light at a discrete detectable wavelength. In certain embodiments, the reference dye and the sensor dye are chemically different dyes. In certain embodiments, the reference dye and the sensor dye are separated in different compartments of the nanoparticle. In certain embodiments, the reference dye is associated (e.g., covalently, e.g., non-covalently) to the nanoparticle core. In certain embodiments, the sensor dye is associated (e.g., covalently, e.g., non-covalently) to the nanoparticle surface.

In certain embodiments, the method comprises determining, via a processor of a computing device, a quantitative map (e.g., a real-time quantitative map) of one or more members selected from the group consisting of tissue perfusion, tissue viability, oxygen/pH status, deep tissue, and tumor volume (e.g., for surgical navigation), based on the detected signals.

In certain embodiments, the map is determined by a ratio of the signal emitted by the sensor dye normalized by the signal emitted by the reference dye.

The one or more detected signals are emitted after the one or more nanoparticles are localized within one or more subcellular compartments (e.g., organelles, such as lysosomes, Golgi, etc.), and/or within/across a blood brain barrier.

In another aspect, the disclosure describes a method for super-resolution imaging (e.g., at a resolution greater than Abbe's diffraction limit) (e.g., using a super-resolution microscope), the method comprising: (a) administering to a tissue of a subject a composition comprising one or more nanoparticles (e.g. photoswitchable nanoparticles), wherein each of the one or more nanoparticles has a diameter from about 1 nm to about 10 nm (e.g., from about 2 nm to about 8 nm); (b) detecting two or more signals emitted by the administered one or more nanoparticles; and (c) graphically rendering, via a processor of a computing device, based on the detected signal, a location of (e.g., a map of) one or more nanoparticles localized within one or more cells of the tissue of the subject.

In certain embodiments, the method is for subcellular, clinical applications, personalized medicine (e.g., coupled with proteomics and/or genomics), and/or for mapping particle distribution and delivery to and/or escape from one or more subcellular compartments (e.g., organelles), and/or a blood brain barrier.

In certain embodiments, the method is for subcellular, clinical applications, personalized medicine (e.g., coupled with proteomics and/or genomics), and/or for mapping particle distribution and delivery to and/or escape from one or more subcellular compartments (e.g., organelles), and/or blood barrier to count numbers of one or more nanoparticles (e.g., dose) delivered to the one or more compartments and/or blood barrier as part of e.g., drug delivery applications or toxicological evaluation.

Each of the one or more nanoparticles operates as a nanosensor for one or more environmental conditions and/or analytes selected from the group consisting of reactive oxygen species (ROS), pH, pH perturbation, iron level, calcium, glutathione, leucine, glutamine, arginine, and other amino acid, wherein each of the one or more or more nanoparticles comprises two or more dyes, the two or more photoluminescent dyes comprising at least one reference dye (e.g., ATTO-647N) and at least one sensor dye (e.g., FITC), and wherein the reference dye exhibits a relatively constant photon emission and the sensor dye exhibits different photon emissions depending on the one more environmental conditions.

In certain embodiments, the nanosensor comprises a pathway inhibitor and/or other immune modulator (and, optionally, a targeting agent).

In certain embodiments, each dye comprises an independently-detectable fluorophore. In certain embodiments, each dye emits light at a discrete detectable wavelength. In certain embodiments, the reference dye and the sensor dye are chemically different dyes. In certain embodiments, the reference dye and the sensor dye are separated in different compartments of the nanoparticle. In certain embodiments, the reference dye is associated (e.g., covalently, e.g., non-covalently) to the nanoparticle core. In certain embodiments, the sensor dye is associated (e.g., covalently, e.g., non-covalently) to the nanoparticle surface.

The method comprises detecting two or more signals from the photon emissions from the reference dye and sensor dye emitted by the administered nanoparticles, wherein the two or more signals indicate one or more environmental conditions and/or analytes (e.g., on a subcellular level) selected from the group consisting of reactive oxygen species (ROS), pH, pH perturbation, iron level, calcium, glutathione, leucine, glutamine, arginine, and other amino acid of the tissue; and in certain embodiments, determining, via a processor of a computing device, a map (e.g., a quantitative map, e.g., a real-time quantitative map) of one or more members selected from the group consisting of tissue perfusion, tissue viability, oxygen/pH status, deep tissue, and tumor volume (e.g., for surgical navigation, e.g., for personalized medicine, e.g., for determining dosing limits for drug delivery), based on the detected signals.

In certain embodiments, the method comprises identifying a location of (e.g., a map of) one or more nanoparticles localized within one or more cells of the tissue of the subject. The one or more nanoparticles are localized within one or more cellular compartments (e.g., subcellular organelles), ., , and/or within/across the blood brain barrier.

In certain embodiments, the map is determined by a ratio of the signal emitted by the sensor dye normalized by the signal emitted by the reference dye.

In certain embodiments, the method comprises displaying, via a graphical display, the map (e.g., co-registered with an *ex vivo* super-resolution microscopy of tissue section, thereby facilitating surgical treatment/-management decision making).

In certain embodiments, the method comprises administering the one or more nanoparticles to the subject for accumulation at sufficiently high concentration in tumor tissue to induce ferroptosis (e.g., ferroptotic cell death involving iron-dependent necrosis or reactive oxygen species-dependent necrosis), as part of a combination therapy. In certain embodiments, the combination therapy further comprises administering to the subject (i) one or more standard-of-care ICB antibodies and/or one or more small molecule inhibitors; or (ii) one or more standard-of-care anti-androgen receptor therapeutics and/or a hypoxia-activated prodrug.

In certain embodiments, the method comprises monitoring and/or disease tracking (e.g., continuously, e.g., in real-time, e.g., during surgery), via a detector, responses of the subject to treatment by detecting one or more environmental conditions and/or analytes selected from the group consisting of reactive oxygen species (ROS), pH, pH perturbation, iron level, calcium, glutathione, leucine, glutamine, arginine, and other amino acid via a readout on the detector (e.g., a 2D or 3D map of the detected environmental condition and/or analyte level).

The method comprises identifying the administered one or more nanoparticles in the tissue of the subject at a subcellular level (e.g., an organelle or suborganelle level, e.g. at a resolution near and/or greater than Abbe's diffraction limit).

In certain embodiments, the identifying is (i) for assessment of nanoparticle delivery and/or trafficking and/or (ii) for nanosensor imaging of cancer metabolism and/or therapeutic response and/or progression and/or the one or more environmental conditions (e.g., microenvironment), e.g., thereby informing therapy adjustment. The identifying is (i) for assessment the identifying comprises counting individual nanoparticles, e.g., for assessing a number of one or more nanoparticles localized in one or more subcellular compartments and/or for assessing unanticipated nanoparticle accumulations leading to unwanted events.

In certain embodiments, the method comprises determining, based on the one or more nanoparticles, localized within one or more cells of the tissue of the subject, a dosing limit for drug delivery.

In the invention, the one or more nanoparticles are silica-based. The one or more nanoparticles comprise one or more silica-based nanosensors. In certain embodiments, the one or more nanoparticles comprise one or more silica-based photoswitchable nanoparticles. The one or more nanoparticles comprise: a silica-based core; a fluorescent compound within the core; a silica shell surrounding at least a portion of the core; and an organic polymer attached to the nanoparticle, thereby coating the nanoparticle.

The one or more nanoparticles have an average diameter no greater than about 10 nm (e.g., no greater than about 8 nm).

In certain embodiments, the one or more nanoparticles have an average diameter from about 5 nm to about 7 nm (e.g., about 6 nm).

In certain embodiments, the one or more nanoparticles comprise a member selected from the group consisting of C dots, C' dots, srC' dots, and iC' dots.

In certain embodiments, the nanoparticles comprise from 1 to 60 targeting moieties (e.g., from 1 to 40 targeting moieties, e.g., from 1 to 30 targeting moieties, e.g., from 1 to 20 targeting moieties), wherein the targeting moieties bind to receptors on tumor cells.

In certain embodiments, the administered nanoparticles have a drug (e.g., a chemotherapeutic agent) attached. In certain embodiments, the drug is attached via a linker moiety (e.g., attached covalently or non-covalently).

## Definitions

In order for the present disclosure to be more readily understood, certain terms are first defined below. Additional definitions for the following terms and other terms are set forth throughout the specification.

In this application, the use of "or" means "and/or" unless stated otherwise. As used in this application, the term "comprise" and variations of the term, such as "comprising" and "comprises," are not intended to exclude other additives, components, integers or steps. As used in this application, the terms "about" and "approximately" are used as equivalents. Any numerals used in this application with or without about/approximately are meant to cover any normal fluctuations appreciated by one of ordinary skill in the relevant art. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*"Administration":* The term "administration" refers to introducing a substance into a subject. In general, any route of administration may be utilized including, for example, parenteral (e.g., intravenous), oral, topical, subcutaneous, peritoneal, intraarterial, inhalation, vaginal, rectal, nasal, introduction into the cerebrospinal fluid, or instillation into body compartments. In certain embodiments, administration is oral. Additionally or alternatively, in certain embodiments, administration is parenteral. In certain embodiments, administration is intravenous.

*"Antibody"*: As used herein, the term "antibody" refers to a polypeptide that includes canonical immunoglobulin sequence elements sufficient to confer specific binding to a particular target antigen. Intact antibodies as produced in nature are approximately 150 kD tetrameric agents comprised of two identical heavy chain polypeptides (about 50 kD each) and two identical light chain polypeptides (about 25 kD each) that associate with each other into what is commonly referred to as a "Y-shaped" structure. Each heavy chain is comprised of at least four domains (each about 110 amino acids long)- an amino-terminal variable (VH) domain (located at the tips of the Y structure), followed by three constant domains: $CH_1$, $CH_2$, and the carboxy-terminal $CH_3$ (located at the base of the Y's stem). A short region, known as the "switch", connects the heavy chain variable and constant regions. The "hinge" connects $CH_2$ and $CH_3$ domains to the rest of the antibody. Two disulfide bonds in this hinge region connect the two heavy chain polypeptides to one another in an intact antibody. Each light chain is comprised of two domains - an amino-terminal variable (VL) domain, followed by a carboxy-terminal constant (CL) domain, separated from one another by another "switch". Intact antibody tetramers are comprised of two heavy chain-light chain dimers in which the heavy and light chains are linked to one another by a single disulfide bond; two other disulfide bonds connect the heavy chain hinge regions to one another, so that the dimers are connected to one another and the tetramer is formed. Naturally-produced antibodies are also glycosylated, typically on the $CH_2$ domain. Each domain in a natural antibody has a structure characterized by an "immunoglobulin fold" formed from two beta sheets (e.g., 3-, 4-, or 5-stranded sheets) packed against each other in a compressed antiparallel beta barrel. Each variable domain contains three hypervariable loops known as "complement determining regions" (CDR1, CDR2, and CDR3) and four somewhat invariant "framework" regions (FR1, FR2, FR3, and FR4). When natural antibodies fold, the FR regions form the beta sheets that provide the structural framework for the domains, and the CDR loop regions from both the heavy and light chains are brought together in three-dimensional space so that they create a single hypervariable antigen binding site located at the tip of the Y structure. The Fc region of naturally-occurring antibodies binds to elements of the complement system, and also to receptors on effector cells, including for example effector cells that mediate cytotoxicity. Affinity and/or other binding attributes of Fc regions for Fc receptors can be modulated through glycosylation or other modification. In certain embodiments, antibodies produced and/or utilized in accordance with the present invention include glycosylated Fc domains, including Fc domains with modified or engineered such glycosylation. For purposes of the present invention, in certain embodiments, any polypeptide or complex of polypeptides that includes sufficient immunoglobulin domain sequences as found in natural antibodies can be referred to and/or used as an "antibody", whether such polypeptide is naturally produced (e.g., generated by an organism reacting to an antigen), or produced by recombinant engineering, chemical synthesis, or other artificial system or methodology. In certain embodiments, an antibody is polyclonal; in certain embodiments, an antibody is monoclonal. In certain embodiments, an antibody has constant region sequences that are characteristic of mouse, rabbit, primate, or human antibodies. In certain embodiments, antibody sequence elements are humanized, primatized, chimeric, etc, as is known in the art. Moreover, the term "antibody" as used herein, can refer in appropriate embodiments (unless otherwise stated or clear from context) to any of the art-known or developed constructs or formats for utilizing antibody structural and functional features in alternative presentation. For example, embodiments, an antibody utilized in accordance with the present invention is in a format selected from, but not limited to, intact IgG, IgE and IgM, bi- or multi- specific antibodies (e.g., Zybodies®, etc), single chain Fvs, polypeptide-Fc fusions, Fabs, cameloid antibodies, masked antibodies (e.g., Probodies®), Small Modular Immuno-Pharmaceuticals ("SMIPs™"), single chain or Tandem diabodies (TandAb®), VHHs, Anticalins®, Nanobodies®, minibodies, BiTE®s, ankyrin repeat proteins or DARPINs®, Avimers®, a DART, a TCR-like antibody, Adnectins®, Affilins®, Trans-bodies®, Affibodies®, a TrimerX®, MicroProteins, Fynomers®, Centyrins®, and a KALBITOR®. In certain embodiments, an antibody may lack a covalent modification (e.g., attachment of a glycan) that it would have if produced naturally. In certain embodiments, an antibody may contain a covalent modification (e.g., attachment of a glycan, a payload [e.g., a detectable moiety, a therapeutic moiety, a catalytic moiety, etc], or other pendant group [e.g., poly-ethylene glycol, etc.]).

*"Antibody fragment"*: As used herein, an "antibody fragment" includes a portion of an intact antibody, such as, for example, the antigen-binding or variable region of an antibody. In certain embodiments, the nanoparticles described herein comprise, have attached, and/or have associated therewith one or more antibodies and/or one or more antibody fragments. Examples of antibody fragments include Fab, Fab', F(ab')2, and Fv fragments; triabodies; tetrabodies; linear antibodies; single-chain antibody molecules; and multi specific antibodies formed from antibody fragments. For example, antibody fragments include isolated fragments, "Fv" fragments, consisting of the variable regions of the heavy and light chains, recombinant single chain polypeptide molecules in which light and heavy chain variable regions are connected by a peptide linker ("ScFv proteins"), and minimal recognition units consisting of the amino acid residues that mimic the hypervariable region. In many embodiments, an antibody fragment contains sufficient sequence of the parent antibody of which it is a fragment that it binds to the same antigen as does the parent antibody; in certain embodiments, a fragment binds to the antigen with a comparable affinity to that of the parent antibody and/or competes with the parent antibody for binding to the antigen. Examples of antigen binding fragments of an antibody include, but are not limited to, Fab fragment, Fab' fragment, F(ab')2 fragment, scFv fragment, Fv fragment, dsFv diabody, dAb fragment, Fd' fragment, Fd fragment, and an isolated complementarity determining region (CDR) region. An antigen binding fragment of an antibody may be produced by any means. For example, an antigen binding fragment of an antibody may be enzymatically or chemically produced by fragmentation of an intact antibody and/or it may be recombinantly produced from a gene encoding the partial antibody sequence. Alternatively or additionally, antigen binding fragment of an antibody may be wholly or partially synthetically produced. An antigen binding fragment of an antibody may optionally comprise a single chain antibody fragment. Alternatively or additionally, an antigen binding fragment of an antibody may comprise multiple chains which are linked together, for example, by disulfide linkages. An antigen binding fragment of an antibody may optionally comprise a multimolecular complex. A functional single domain antibody fragment is in a range from about 5 kDa to about 25 kDa, e.g., from about 10 kDa to about 20 kDa, e.g., about 15 kDa; a functional single-chain fragment is from about 10 kDa to about 50 kDa, e.g., from about 20 kDa to about 45 kDa, e.g., from about 25 kDa to about 30 kDa; and a functional fab fragment is from about 40 kDa to about 80 kDa, e.g., from about 50 kDa to about 70 kDa, e.g., about 60 kDa.

*"Associated"*: As used herein, the term "associated" typically refers to two or more entities in physical proximity with one another, either directly or indirectly (e.g., via one or more additional entities that serve as a linking agent), to form a structure that is sufficiently stable so that the entities remain in physical proximity under relevant conditions, e.g., physiological conditions. In certain embodiments, associated moieties are covalently linked to one another. In certain embodiments, associated entities are non-covalently linked. In certain embodiments, associated entities are linked to one another by specific non-covalent interactions (i.e., by interactions between interacting ligands that discriminate between their interaction partner and other entities present in the context of use, such as, for example. streptavidin/avidin interactions, antibody/antigen interactions, *etc.*). Alternatively or additionally, a sufficient number of weaker non-covalent interactions can provide sufficient stability for moieties to remain associated. Exemplary non-covalent interactions include, but are not limited to, electrostatic interactions, hydrogen bonding, affinity, metal coordination, physical adsorption, host-guest interactions, hydrophobic interactions, pi stacking interactions, van der Waals interactions, magnetic interactions, electrostatic interactions, dipole-dipole interactions, *etc.*

*"Agent"*: The term "agent" refers to a compound or entity of any chemical class including, for example, polypeptides, nucleic acids, saccharides, lipids, small molecules, metals, or combinations thereof. In certain embodiments, the nanoparticles described herein comprise, have attached, or have associated therewith one or more agents. As will be clear from context, in certain embodiments, an agent can be or comprise a cell or organism, or a fraction, extract, or component thereof. In certain embodiments, an agent is or comprises a natural product in that it is found in and/or is obtained from nature. In certain embodiments, an agent is or comprises one or more entities that are man-made in that it is designed, engineered, and/or produced through action of the hand of man and/or are not found in nature.

In certain embodiments, an agent may be utilized in isolated or pure form; in certain embodiments, an agent may be utilized in crude form. In certain embodiments, potential agents are provided as collections or libraries, for example that may be screened to identify or characterize active agents within them. Some particular embodiments of agents that may be utilized include small molecules, antibodies, antibody fragments, aptamers, siRNAs, shRNAs, DNA/RNA hybrids, antisense oligonucleotides, ribozymes, peptides, peptide mimetics, peptide nucleic acids, small molecules, etc. In certain embodiments, an agent is or comprises a polymer. In certain embodiments, an agent contains at least one polymeric moiety. In certain embodiments, an agent comprises a therapeutic, diagnostic and/or drug.

*"Biocompatible"*: The term "biocompatible", as used herein is intended to describe materials that do not elicit a substantial detrimental response in vivo. In certain embodiments, the materials are "biocompatible" if they are not toxic to cells. In certain embodiments, materials are "biocompatible" if their addition to cells *in vitro* results in less than or equal to 20% cell death, and/or their administration *in vivo* does not induce inflammation or other such adverse effects. In certain embodiments, materials are biodegradable.

*"Biodegradable"*: As used herein, "biodegradable" materials are those that, when introduced into cells, are broken down by cellular machinery (*e.g.*, enzymatic degradation) or by hydrolysis into components that cells can either reuse or dispose of without significant toxic effects on the cells. In certain embodiments, components generated by breakdown of a biodegradable material do not induce inflammation and/or other adverse effects *in vivo*. In certain embodiments, biodegradable materials are enzymatically broken down. Alternatively or additionally, in certain embodiments, biodegradable materials are broken down by hydrolysis. In certain embodiments, biodegradable polymeric materials break down into their component polymers. In certain embodiments, breakdown of biodegradable materials (including, for example, biodegradable polymeric materials) includes hydrolysis of ester bonds. In certain embodiments, breakdown of materials (including, for example, biodegradable polymeric materials) includes cleavage of urethane linkages.

*"Cancer"*: As used herein, the term "cancer" refers to a malignant neoplasm or tumor (Stedman's Medical Dictionary, 25th ed.; Hensly ed.; Williams & Wilkins: Philadelphia, 1990). Exemplary cancers include, but are not limited to, brain cancer (e.g., meningioma, glioblastomas, glioma (e.g., astrocytoma, oligodendroglioma), medulloblastoma), prostate cancer, melanoma, breast cancer, gynecological malignancies, colorectal cancers.

*"Carrier"*: As used herein, "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin. In certain embodiments, compositions described herein (e.g., compositions administered to a subject, e.g., compositions comprising a nanoparticle described herein) comprise a carrier.

*"Detector":* As used herein, the term "detector" includes any detector of electromagnetic radiation including, but not limited to, CCD camera, photomultiplier tubes, photodiodes, and avalanche photodiodes.

*"Environmental conditions":* As used herein, the term "environmental conditions" refers to any environmental conditions that can be determined in one or more subcellular compartments (e.g., organelles, such as lysosomes, Golgi, etc) and/or structures e.g., microtubules). The environmental conditions can be determined within/across multi-compartment tissues (e.g., blood brain barrier, e.g., barriers defining compartments within normal organs, e.g., kidney (e.g., kidney tissue and/or renal tissue)). Environmental conditions include pH levels (and/or pH perturbations), oxygen (e.g., reactive oxygen species (ROS)) levels, hydrophobic or hydrophilic states, the presence of and concentration of ions (e.g., potassium, phosphate, sodium, calcium, copper, magnesium, chromium, chloride, fluoride, iron), heavy metals (e.g., cadmium, zinc, lead, selenium, mercury, nickel), biomolecular substances (e.g., vitamins, amino acids (e.g., leucine, glutamine, arginine)), and the like.

*"Fluorescent dye":* The nanoparticle comprises or has attached a fluorescent dye comprising one or more fluorophores. Fluorophores comprise fluorochromes, fluorochrome quencher molecules, any organic or inorganic dyes, metal chelates, or any fluorescent enzyme substrates, including protease activatable enzyme substrates. In certain embodiments, fluorophores comprise long chain carbophilic cyanines. In other embodiments, fluorophores comprise DiI, DiR, DiD, and the like. Fluorochromes comprise far red, and near infrared fluorochromes (NIRF). Fluorochromes include but are not limited to a carbocyanine and indocyanine fluorochromes. In certain embodiments, imaging agents comprise commercially available fluorochromes including, but not limited to Cy5.5, Cy5 and Cy7 (GE Healthcare); AlexaFlour660, AlexaFlour680, AlexaFluor750, and AlexaFluor790 (Invitrogen); VivoTag680, VivoTag-S680, and VivoTag-S750 (VisEn Medical); Dy677, Dy682, Dy752 and Dy780 (Dyomics); DyLight547, DyLight647 (Pierce); HiLyte Fluor 647, HiLyte Fluor 680, and HiLyte Fluor 750 (AnaSpec); IRDye 800CW, IRDye 800RS, and IRDye 700DX (Li-Cor); and ADS780WS, ADS830WS, and ADS832WS (American Dye Source) and Kodak X-SIGHT 650, Kodak X-SIGHT 691, Kodak X-SIGHT 751 (Carestream Health).

*"In vitro":* The term *"in vitro"* as used herein refers to events that occur in an artificial environment, e.g., in a test tube or reaction vessel, in cell culture, etc., rather than within a multi-cellular organism.

*"In vivo":* As used herein *"in vivo"* refers to events that occur within a multi-cellular organism, such as a human and a non-human animal. In the context of cell-based systems, the term may be used to refer to events that occur within a living cell (as opposed to, for example, *in vitro* systems).

*"Imaging agent":* The term "imaging agent" as used herein refers to any element, molecule, functional group, compound, fragments thereof or moiety that facilitates detection of an agent (e.g., a polysaccharide nanoparticle) to which it is joined. In certain embodiments, the nanoparticles described herein comprise, have attached, and/or have associated therewith one or more imaging agents. Examples of imaging agents include, but are not limited to: various ligands, radionuclides (e.g., $^3$H, $^{14}$C, $^{18}$F, $^{19}$F, $^{32}$P, $^{35}$S, $^{135}$I, $^{125}$I, $^{123}$I, $^{64}$Cu, $^{187}$Re, $^{111}$In, $^{90}$Y, $^{99m}$Tc, $^{177}$Lu, $^{89}$Zr etc.), fluorescent dyes (for specific exemplary fluorescent dyes, see below), chemiluminescent agents (such as, for example, acridinum esters, stabilized dioxetanes, and the like), bioluminescent agents, spectrally resolvable inorganic fluorescent semiconductors nanocrystals (i.e., quantum dots), metal nanoparticles (e.g., gold, silver, copper, platinum, etc.) nanoclusters, paramagnetic metal ions, enzymes (for specific examples of enzymes, see below), colorimetric labels (such as, for example, dyes, colloidal gold, and the like), biotin, dioxigenin, haptens, and proteins for which antisera or monoclonal antibodies are available.

*"Image":* The term "image", as used herein, is understood to mean a visual display or any data representation that may be interpreted for visual display. For example, a three-dimensional image may include a dataset of values of a given quantity that varies in three spatial dimensions. A three-dimensional image (e.g., a three-dimensional data representation) may be displayed in two-dimensions (e.g., on a two-dimensional screen, or on a two-dimensional printout). In certain embodiments, the term "image" may refer to, for example, to a multi-dimensional image (e.g., a multi-dimensional (e.g., four dimensional) data representation) that is displayed in two-dimensions (e.g., on a two-dimensional screen, or on a two-dimensional printout). The term "image" may refer, for example, to an optical image, an x-ray image, an image generated by: positron emission tomography (PET), magnetic resonance, (MR) single photon emission computed tomography (SPECT), and/or ultrasound, and any combination of these.

*"Nanoparticle ":* As used herein, the term "nanoparticle" refers to a particle having a diameter of less than 1000 nanometers (nm). A nanoparticle has a diameter of less than 300 nm, as defined by the National Science Foundation. A nanoparticle has a diameter of less than 100 nm as defined by the National Institutes of Health. In the invention. the nanoparticle (inclusive of any ligands or other attached or associated species), is no greater than about 10 nm in diameter. In certain embodiments, nanoparticles are micelles in that they comprise an enclosed compartment, separated from the bulk solution by a micellar membrane, typically comprised of amphiphilic entities which surround and enclose a space or compartment (e.g., to define a lumen). In certain embodiments, a micellar membrane is comprised of at least one polymer, such as for example a biocompatible and/or biodegradable polymer.

"*Peptide*" or "*Polypeptide*": The term "peptide" or "polypeptide" refers to a string of at least two (e.g., at least three) amino acids linked together by peptide bonds. In certain embodiments, a polypeptide comprises naturally-occurring amino acids; alternatively or additionally, in certain embodiments, a polypeptide comprises one or more non-natural amino acids (i.e., compounds that do not occur in nature but that can be incorporated into a polypeptide chain; see, for example, http://www.cco.caltech.edu/~dadgrp/Unnatstruct.gif, which displays structures of non-natural amino acids that have been successfully incorporated into functional ion channels) and/or amino acid analogs as are known in the art may alternatively be employed). In certain embodiments, one or more of the amino acids in a protein may be modified, for example, by the addition of a chemical entity such as a carbohydrate group, a phosphate group, a farnesyl group, an isofamesyl group, a fatty acid group, a linker for conjugation, functionalization, or other modification, *etc.*

"*Protein*": As used herein, the term "protein" refers to a polypeptide (i.e., a string of at least 3-5 amino acids linked to one another by peptide bonds). Proteins may include moieties other than amino acids (e.g., may be glycoproteins, proteoglycans, etc.) and/or may be otherwise processed or modified. In certain embodiments "protein" can be a complete polypeptide as produced by and/or active in a cell (with or without a signal sequence); in certain embodiments, a "protein" is or comprises a characteristic portion such as a polypeptide as produced by and/or active in a cell. In certain embodiments, a protein includes more than one polypeptide chain. For example, polypeptide chains may be linked by one or more disulfide bonds or associated by other means. In certain embodiments, proteins or polypeptides as described herein may contain L-amino acids, D-amino acids, or both, and/or may contain any of a variety of amino acid modifications or analogs known in the art. Useful modifications include, e.g., terminal acetylation, amidation, methylation, etc. In certain embodiments, proteins or polypeptides may comprise natural amino acids, non-natural amino acids, synthetic amino acids, and/or combinations thereof. In certain embodiments, proteins are or comprise antibodies, antibody polypeptides, antibody fragments, biologically active portions thereof, and/or characteristic portions thereof.

"*Pharmaceutical composition*": As used herein, the term "pharmaceutical composition" refers to an active agent, formulated together with one or more pharmaceutically acceptable carriers. In certain embodiments, active agent is present in unit dose amount appropriate for administration in a therapeutic regimen that shows a statistically significant probability of achieving a predetermined therapeutic effect when administered to a relevant population. In certain embodiments, pharmaceutical compositions may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream, or foam; sublingually; ocularly; transdermally; or nasally, pulmonary, and to other mucosal surfaces.

"*Radiolabel*": As used herein, "radiolabel" refers to a moiety comprising a radioactive isotope of at least one element. Exemplary suitable radiolabels include but are not limited to those described herein. In certain embodiments, a radiolabel is one used in positron emission tomography (PET). In certain embodiments, a radiolabel is one used in single-photon emission computed tomography (SPECT). In certain embodiments, radio-isotopes comprise $^{99m}$Tc, $^{111}$In, $^{64}$Cu, $^{67}$Ga, $^{186}$Re, $^{188}$Re, $^{153}$Sm, $^{177}$Lu, $^{67}$Cu, $^{123}$I, $^{124}$I, $^{125}$I, $^{11}$C, $^{13}$N, $^{15}$O, $^{18}$F, $^{186}$Re, $^{188}$Re, $^{153}$Sm, $^{166}$Ho, $^{177}$Lu, $^{149}$Pm, $^{90}$Y, $^{213}$Bi, $^{103}$Pd, $^{109}$Pd, $^{159}$Gd, $^{140}$La, $^{198}$Au, $^{199}$Au, $^{169}$Yb, $^{175}$Yb, $^{165}$Dy, $^{166}$Dy, $^{67}$Cu, $^{105}$Rh, $^{111}$Ag, $^{89}$Zr, $^{225}$Ac, and $^{192}$Ir.

"*Redox status*": As used herein, the term "redox status" relates to the redox status of a cell (e.g., within and/or derived from a subject), and describes its oxidation-reduction potential (e.g., the potential of the cell to lose electrons (oxidation) versus its potential to gain electrons (reduction)). In certain embodiments, the present disclosure describes in vivo and in vitro testing with nanoparticles (e.g., nanosensors) to determine the redox status of cells. In certain embodiments, the described systems and methods can measure changes in the redox status of a cell. In certain embodiments, glutathione is measured.

"*Sensor*": As used herein, the term "sensor" includes any sensor of electromagnetic radiation including, but not limited to, CCD camera, photomultiplier tubes, photodiodes, and avalanche photodiodes, unless otherwise evident from the context.

"*Subject*": As used herein, the term "subject" includes humans and mammals (e.g., mice, rats, pigs, cats, dogs, and horses). In many embodiments, subjects are mammals, particularly primates, especially humans. In certain embodiments, subjects are livestock such as cattle, sheep, goats, cows, swine, and the like; poultry such as chickens, ducks, geese, turkeys, and the like; and domesticated animals particularly pets such as dogs and cats. In certain embodiments (e.g., particularly in research contexts) subject mammals will be , for example, rodents (e.g., mice, rats, hamsters), rabbits, primates, or swine such as inbred pigs and the like.

*"Substantially"*: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*"Super-resolution microscopy"*: As used herein, the term "super-resolution microscopy" refers to microscopy in which a resolution is achieved that is better than (with lower nm value than) that achievable by conventional microscopy. In certain cases, super-resolution microscopy achieves a resolution of 200 nm or less (where a lower value indicates higher resolution), e.g., 175 nm or less, e.g., 150 nm or less, e.g., 125 nm or less, e.g., 120 nm or less, e.g., 110 nm or less, e.g., 100 nm or less, e.g., 75 nm or less, e.g., 50 nm or less. In certain cases, super-resolution microscopy achieves a higher resolution than that imposed by the diffraction limit (e.g., Abbe's diffraction limit). *"Targeting agent"*: Non-limiting examples of targeting agents (e.g., ligands attached to nanoparticles that cause the nanoparticles to accumulate in or near (and/or be driven to) a particular cell type, tissue type, analyte, or other target) include, for example, a targeting peptide, or antibody fragment. In certain embodiments, the nanoparticles described herein comprise one or more targeting agents. In certain embodiments, the targeting agent comprises a targeting peptide (e.g., RGD, e.g., cRGD, e.g., an analog of RGD, e.g., alphaMSH, e.g., any peptide known to be immunomodulatory and anti-inflammatory in nature). In certain embodiments, the targeting agent comprises an antibody fragment, e.g., wherein the antibody fragment is in a range from about 5 kDa to about 25 kDa (e.g., from about 10 kDa to about 20 kDa, e.g., about 15 kDa) (e.g., wherein the antibody fragment comprises a functional single domain antibody fragment). In certain embodiments, the targeting agent comprises an antibody fragment, and wherein the antibody fragment is from about 20 kDa to about 45 kDa (e.g., from about 25 kDa to about 30 kDa) (e.g., wherein the antibody fragment comprises a functional single chain antibody fragment). In certain embodiments, the targeting agent comprises an antibody fragment, and wherein the antibody fragment is from about 40 kDa to about 80 kDa (e.g., from about 50 kDa to about 70 kDa, e.g., about 60 kDa) (e.g., wherein the antibody fragment comprises a functional fab fragment).

*"Therapeutic agent"*: As used herein, the phrase "therapeutic agent" refers to any agent that has a therapeutic effect and/or elicits a desired biological and/or pharmacological effect, when administered to a subject. In certain embodiments, the therapeutic agent comprises a drug, e.g., a chemotherapy drug (e.g., sorafenib, paclitaxel, docetaxel, MEK162, etoposide, lapatinib, nilotinib, crizotinib, fulvestrant, vemurafenib, bexorotene, and/or camptotecin).

*"Therapeutically effective amount"*: as used herein, "therapeutically effective amount" refers to an amount that produces the desired effect for which it is administered. In certain embodiments, the term refers to an amount that is sufficient, when administered to a population suffering from or susceptible to a disease, disorder, and/or condition in accordance with a therapeutic dosing regimen, to treat the disease, disorder, and/or condition. In certain embodiments, a therapeutically effective amount is one that reduces the incidence and/or severity of, and/or delays onset of, one or more symptoms of the disease, disorder, and/or condition. Those of ordinary skill in the art will appreciate that the term *"therapeutically effective amount"* does not in fact require successful treatment be achieved in a particular individual. Rather, a therapeutically effective amount may be that amount that provides a particular desired pharmacological response in a significant number of subjects when administered to patients in need of such treatment. In certain embodiments, reference to a therapeutically effective amount may be a reference to an amount as measured in one or more specific tissues (e.g., a tissue affected by the disease, disorder or condition) or fluids (e.g., blood, saliva, serum, sweat, tears, urine, etc.). Those of ordinary skill in the art will appreciate that, in certain embodiments, a therapeutically effective amount of a particular agent or therapy may be formulated and/or administered in a single dose. In certain embodiments, a therapeutically effective agent may be formulated and/or administered in a plurality of doses, for example, as part of a dosing regimen.

*"Treatment"*: As used herein, the term "treatment" (also "treat" or "treating") refers to any administration of a substance that partially or completely alleviates, ameliorates, relives, inhibits, delays onset of, reduces severity of, and/or reduces incidence of one or more symptoms, features, and/or causes of a particular disease, disorder, and/or condition. Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. In certain embodiments, treatment may be of a subject who has been diagnosed as suffering from the relevant disease, disorder, and/or condition. In certain embodiments, treatment may be of a subject known to have one or more susceptibility factors that are statistically correlated with increased risk of development of the relevant disease, disorder, and/or condition. In certain embodiments, treatment comprises delivery of therapeutics, including but not limited to, small molecule delivery, radiotherapy, immunotherapy, intrinsic therapeutic properties (e.g., ferroptosis), and particle-driven regulation of the tumor microenvironment. In certain embodiments, therapeutics are attached to particles, such as those described herein.

Drawings are presented herein for illustration purposes, not for limitation.

## Brief Description of Drawings

The foregoing and other objects, aspects, features, and advantages of the present disclosure will become more apparent and better understood by referring to the following description taken in conduction with the accompanying drawings, in which:

FIG. 1 is a block diagram of an example network environment for use in the methods and systems for analysis of spectrometry data, according to an illustrative embodiment.

FIG. 2 is a block diagram of an example computing device and an example mobile computing device, for use in illustrative embodiments of the invention.

FIGS. 3A-3B show images of melanoma-targeted C'dots that efficiently kill B16 melanoma cells, according an embodiment described by the present disclosure. FIGS. 3A and 3B show an image depicting that treatment of B16 mouse melanoma cells with αMSH-C' dots leads to ferroptosis (middle panel, dead cells indicated with Sytox green staining) that propagates and kills the cell population. Cell nuclei are pseudocolored to indicate the timing of cell death.

FIGS. 4A and 4B show images and a plot that depict that C' dot nanoparticles induce ferroptosis that spreads through cell populations and kills prostate cancer in combination with enzalutamide.

FIGS. 5A-5D show images (FIGS. 5A-5C) and a plot (FIG. 5D) depicting super-resolution imaging of cRGDY-PEG-C dot lysosomal uptake.

FIGS. 6A-C show graphs (FIGS. 6A-6B) and images (FIG. 6C) depicting nanosensors for quantification of intracellular pH.

## Detailed Description

Throughout the description, where compositions are described as having, including, or comprising specific components, or where methods are described as having, including, or comprising specific steps, it is contemplated that, additionally, there are compositions of the present invention that consist essentially of, or consist of, the recited components, and that there are methods according to the present invention that consist essentially of, or consist of, the recited processing steps.

It should be understood that the order of steps or order for performing certain action is immaterial so long as the invention remains operable. Moreover, two or more steps or actions may be conducted simultaneously.

The mention herein of any publication, for example, in the Background section, is not an admission that the publication serves as prior art with respect to any of the claims presented herein. The Background section is presented for purposes of clarity and is not meant as a description of prior art with respect to any claim.

Described herein are imaging systems and methods that provide super-resolution visualization of perfusion at the cellular level (e.g., using super-resolution microscopy, nanosensors, and/or photoswitchable nanoparticles). In particular, the systems and methods enable a medical practitioner to assess the nature of (e.g., cancerous, non-cancerous) and/or viability of a region of remaining tissue during or after tumor excision or other tissue removal surgery. The super-resolution capabilities, for example, of the technology enable the practitioner to remove less normal tissue surrounding a tumor during surgery, with lower risk of recurrence. The technology also helps assess the viability of a graft (e.g., implanted tissue at a surgical site) peri-operatively (pre-, intra-, and post-surgery).

Imaging of sub-wavelength structures is challenging because of Abbe's diffraction limit, as follows:

$$d = \frac{\lambda}{2n \sin \theta} = \frac{\lambda}{2NA},$$

where λ is the wavelength of illuminating light traveling in a medium with refractive index n and converging to a spot with half-angle θ, and NA is the numerical aperture, which can reach about 1.4-1.6, such that Abbe's diffraction limit is about λ/2.8. To increase resolution, shorter wavelengths can be used; however, these techniques are expensive and may damage tissue.

In certain embodiments, the methods can be used in the detection, characterization and/or determination of the localization of a disease, especially early disease, the severity of a disease or a disease-associated condition, the staging of a disease, and monitoring and guiding various therapeutic interventions, such as surgical procedures, and monitoring and/or development of drug therapy and delivery, including cell based therapies.

In certain embodiments, the technology provides apparatus, compositions, systems, and methods for super-resolution visualization of transport at the cellular level. For example, the technology enables a medical practitioner to assess the nature of (e.g., cancerous, non-cancerous) and/or viability of a region (e.g., a limb) of remaining tissue during or after tumor excision or other tissue removal surgery (e.g., and determine whether the region is properly utilizing oxygen) (e.g., in a pre-operative setting). The super-resolution capabilities of the technology, for example, enable the practitioner to remove less normal tissue surrounding a tumor during surgery, with lower risk of recurrence. The technology uses a sensor that is ratiometric (e.g., uses a baseline reference), and also helps assess the viability of a graft (e.g., implanted tissue at a surgical site) peri-operatively (pre-, intra-, and post-surgery).

Moreover, the technology allows a medical practitioner to monitor and/or deliver treatment in a non-surgical setting. For example, the medical practitioner is able to implant a graft and use a sensor to monitor the area. The sensor can be provided directly or via a device (e.g., transdermal) (e.g., by a catheter). The sensor may be responsive to the environment and provides controlled release of a composition (e.g., a substrate, e.g., a drug) for monitoring and/or treatment of the area of interest.

The technology also provides the ability to monitor a local microenvironment (e.g., where a medical practitioner has implanted cells/clusters of cells), for example, in diseases such as bone marrow transplants, kidney transplants, and liver transplants. By using the compositions described herein (e.g., photoswitchable nanoparticles), monitoring of drug delivery down to 10 nm or below can be achieved.

*Nanoparticles*

In certain embodiments, one or more nanoparticles are selected from the photoswitchable nanoparticles described by Kohle et al., "Sulfur- or Heavy Atom-Containing Nanoparticles, Methods or Making the Same, and Uses Thereof," in International Application No. PCT/US18/26980 filed on April 10, 2018, the photoluminescent silica-based sensors described by Burns et al. "Photoluminescent Silica-Based Sensors and Methods of Use" in US Patent No. US 8,084,001 B1, and/or the nanoparticles described by Bradbury et al., "Ultrasmall Nanoparticles Labeled with Zirconium-89 and Methods Thereof," International Patent Application No. PCT/US18/33098, filed on May 17, 2018. In certain embodiments, the nanoparticle is a modification or combination of any of such compositions.

In certain embodiments, the nanoparticles described herein are utilized in systems and methods for mapping surgical margins with cellular-level resolution. In certain embodiments, the described systems and methods are used as super-resolution imaging tools (e.g., toggling super-resolution microscopy and/or targeted nanoparticles), and/or in interactive 3D ultra-high resolution displays of remnant cancer cells.

In certain embodiments, the nanoparticles described herein are utilized in systems and methods for perioperative metabolic sensing to predict response and tissue viability. For example, the disclosed nanoparticles can be used as metabolic & responsive nanosensors that monitor/treat derangements in pH, oxygen or continuous 3D super resolution real-time maps of tissue metabolism (hypoxia).

In certain embodiments, the nanoparticles described herein are utilized in systems and methods for image-guided treatment planning for surgery, IR, and radiotherapy. For example, apps driving multi-functional imaging, surgical navigation, augmented reality; and/or highly Interactive iSurgery Tablet Tools can be used in the disclosed systems and methods.

In certain embodiments, the nanoparticles described herein are utilized in systems and methods for self-programmable nanotherapies (clinical care) and/or in systems, methods, and compositions for controlled delivery and release of metabolites/nanomedicines to regulate tumor microenvironment or cellular viability. For example, the nanoparticles can be used as delivery/release of pathway inhibitors and/or other immune modulators on the basis of sensing pH, ROS, etc., for reprogramming tumor-infiltrating macrophages, effector cells, and/.or improving response to T-cell checkpoint immunotherapy.

In certain embodiments, the nanoparticles described herein are utilized in systems and methods for "on-demand" treatment and monitoring during surgery and/or for continuous, nano-sensor-driven patient monitoring and 'on-demand' treatment in surgical settings. For example, the nanoparticles can be used in systems and methods for continuous, real-time monitoring of surgical/adjuvant treatment responses that can activate metabolic 'triggers or switches' to adjust for deficient or excess metabolite levels by releasing substrates at the tissue/cellular level or adsorbing excess metabolites. As another example, the nanoparticles can be used as a surgical tool for predicting post-operative recovery.

In certain embodiments, the nanoparticles described herein are utilized in systems and methods for combined treatment and metabolic sensing and/or for performing real-time quantitative assays of metabolic derangements and fluxes at subcellular resolution (e.g., organelles vs. cytosol) in cancerous cells and tissues. For example, the disclosed system and methods can be performed for testing/monitoring nanomedicine efficacy and/or for detecting ROS, lipid peroxidation, pH perturbations, iron levels, calcium, amino acids, etc., to monitor cancer cell state and responses to targeted therapies in specific organelle compartments (lysosome, mitochondria, plasma membrane, nucleus). Moreover, in certain embodiments, the disclosed system and methods can be used for detection of cell death/other metabolic changes associated with growth arrest, senescence, cell polarization or differentiation, or other altered cell phenotypes, and/or for continuous kinetic monitoring of metabolic species within the cell: oxygen (ROS), pH, amino acids (e.g., leucine, glutamine, arginine), iron, glutathione, etc. to monitor concentration changes within the cell to provide real-time readouts of metabolic state and treat substrate/metabolite derangements in organelles to control/prevent cell growth, division, translation, oncogenic signaling, etc.

In certain embodiments, the nanoparticles described herein are utilized in systems and methods for super-resolution imaging of nanoparticles and/or for tracking targeted drug and/or metabolite delivery using photoswitchable nanoparticles. For example, the disclosed system and methods can be used for tracking to subcellular compartments in human cancers, e.g., in perioperative settings (*in situ* and/or *ex vivo*). As another example, the disclosed system and methods can be used for performing 'optical biopsies' and super-resolution nanoscopy during surgery (e.g., using artificial neural networks/machine learning).

In certain embodiments, the nanoparticles described herein are utilized in systems and methods for 3D infrared, real-time imaging and/or systems and methods for 3D infrared real-time imaging for direct nerve visualization during surgery and deep tissue endoscopy *in situ.* For example, the disclosed system and methods can be used for seeing nerves through fat, and real-time tumor margin assessment. In certain embodiments, cellular resolution optical fiber-based devices (multi-photon endoscopes) for virtual biopsies, biopsy sampling, and surgical margin assessments *in situ* are used in the disclosed systems and methods.

In certain embodiments, the nanoparticles described herein are utilized in systems and methods for artificial neural network (ANN)/machine learning in nanomedicine and/or ANN-based systems and methods for real-time imaging and data analysis of targeted nanomedicine treatment. For example, the disclosed systems and methods can be used (i) to monitor, design, and/or administer a targeted nanomedicine treatment plan, (ii) to elucidate complexities of metabolic networks and design/modify a targeted nanomedicine treatment plan, and/or (iii) to perform multi-photon endoscopy and/or cellular-resolution optical fiber-based devices for virtual biopsies, biopsy sampling, and surgical margin assessments *in situ.*

In certain embodiments, the nanoparticles described herein are utilized in systems and methods for app-driven (e.g., iPad) real-time surgical imaging systems and/or for super-resolution, interactive, multifunctional imaging, surgical navigation, and cancer treatment planning. For example, the disclosed systems and methods describe real time mapping of (i) tissue perfusion, viability, oxygen/pH status, (ii) deep tissues, (iii) tumor volumes for surgical navigation, and (iv) treatment planning volumes for perioperative high-precision therapies. In alternative embodiments, the disclosed systems and methods describe treatment planning volumes from pre-operative imaging (CT, etc.) co-registered with augmented reality-generated images projected over the operative bed to permit direct application of tumor margin mapping). Moreover, treatment planning volumes from pre-operative imaging (CT, etc.) can be seamlessly co-registered with augmented reality-generated images projected over the operating bed to permit direct application of radioimmunotherapy or high-energy laser-driven x-ray fluorescence therapy, and/or direct application of radio/immuno-therapy or high-energy laser-driven x-ray fluorescence (XRF) therapy for enhancing response, achieving local tumor control, and/or ablating remnant disease (e.g., maps can be overlaid for real-time treatment planning in the operating room).

In certain embodiments, the nanoparticles described herein are utilized in systems and methods for mapping of surgical margins (e.g., with cellular-level precision using super-resolution imaging). For example, co-registration of *in vivo* and *ex vivo* images with *ex vivo* super-resolution microscopy of tissue section to facilitate 'on the fly' surgical treatment/management decision-making can be performed (e.g., to transform pathological assessments to imaging-driven assessments obtained in surgical settings (i.e., in lieu of frozen sections)). Moreover, in certain embodiments, seamless co-registration can be performed via (i) fiducial markers placed along resection margins, (ii) tomographic imaging of lesions *in-situ* and *ex-vivo,* (iii) *ex-vivo* sectioning of lesion specimens, and (iv) co-registration of sectional images with *in situ* tomographic images for accurate surgical margin assessments.

In certain embodiments, the nanoparticle comprises silica, polymer (e.g., poly(lactic-co-glycolic acid) (PLGA)), biologics (e.g., protein carriers), and/or metal (e.g., gold, iron).

The silica-based nanoparticle platform comprises ultrasmall nanoparticles or "C dots," which are fluorescent, organo-silica core shell particles that have diameters controllable down to the sub-10 nm range with a range of modular functionalities. C dots are described by U.S. Patent No. 8298677 B2 "Fluorescent silica-based nanoparticles", U.S. Publication No. 2013/0039848 A1 "Fluorescent silica-based nanoparticles", and U.S. Publication No. US 2014/0248210 A1 "Multimodal silica-based nanoparticles". Incorporated into the silica matrix of the core are near-infrared dye molecules, such as Cy5.5, which provides its distinct optical properties. Surrounding the core is a layer or shell of silica. The silica surface is covalently modified with silyl-polyethylene glycol (PEG) groups to enhance stability in aqueous and biologically relevant conditions. These particles have been evaluated *in vivo* and exhibit excellent clearance properties owing largely to their size and inert surface. Among the additional functionalities incorporated into C dots are chemical sensing, non-optical (PET) image contrast and *in vitro/in vivo* targeting capabilities, which enable their use in visualizing lymph nodes for surgical applications, and melanoma detection in cancer.

C dots provide a unique platform for drug delivery due to their physical properties as well as demonstrated human *in vivo* characteristics. These particles are ultrasmall and benefit from EPR effects in tumor microenvironments, while retaining desired clearance and pharmacokinetic properties. To this end, in certain embodiments, drug constructs are covalently attached to C dots (or other nanoparticles). C dot-based nanoparticle systems for drug delivery provide good biostability, minimize premature drug release, and exhibit controlled release of the bioactive compound. In certain embodiments, peptide-based linkers are used for nanoparticle drug conjugates ("NDCs") and other applications described herein and by Bradbury et al. in U.S. Publication No. US2015/0343091 A1 "Nanoparticle Drug Conjugates".

These linkers, in the context of antibodies and polymers, are stable both *in vitro* and *in vivo,* with highly predictable release kinetics that rely on enzyme catalyzed hydrolysis by lysosomal proteases. For example, cathepsin B, a highly expressed protease in lysosomes, can be utilized to facilitate drug release from macromolecules. By incorporating a short, protease sensitive peptide between the macromolecular backbone and the drug molecule, controlled release of the drug can be obtained in the presence of the enzyme.

In certain embodiments, the nanoparticle is a photoswitchable nanoparticle as described by Kohle et al., "Sulfur- or Heavy Atom-Containing Nanoparticles, Methods or Making the Same, and Uses Thereof," in International Application No. PCT/US18/26980 filed on April 10, 2018.

Kohle et al. describes that the general principle of super-resolution microscopy is to localize the origin of an emitting source by activating only one point-like emitting source within a diffraction-limited area at a time, while other emitters remain in a dark state. Repeated photoswitching and localizing of different fluorophores eventually resolves spatial features below the diffraction limit. To this end, Kohle et al. describes molecular engineering of ultrasmall sub-10nm fluorescent silica probes by tuning the fluorescent properties of the silica particles (e.g., by altering the chemical composition of the silica network) to produce photoswitching behavior. For instance, by strategically altering the precise chemical environment around the covalently bound dye inside the silica network, Kohle et al. shows that intrinsic electronic properties of organic dyes leading to different photonic behavior as compared to their counterparts in solution.

In certain embodiments, an aluminosilicate nanoparticle comprises various amounts of silicon atoms and aluminum atoms. In certain embodiments, an aluminosilicate nanoparticle comprises 0-30 at.% (at.%= atomic percent) (relative to Si) aluminum atoms, including all 0.1 at.% values and ranges therebetween. In an example, an aluminosilicate nanoparticle comprises 1-20 at.% (relative to Si) aluminum atoms. In another example, an aluminosilicate nanoparticle comprises 5-15 at.% (relative to Si) aluminum atoms. In certain embodiments, silica nanoparticles or aluminosilicate nanoparticles comprise one or more sulfur atoms. Such nanoparticles are referred to herein as srC' dots. The sulfur atoms are covalently bonded to the silica network or aluminosilicate network. Sulfur atoms can be incorporated into a silica or aluminosilicate nanoparticle using a sulfur-containing precursor in the synthesis of the nanoparticle.

In certain embodiments, a nanoparticle comprises various amounts of sulfur atoms. For example, a silica or aluminosilicate nanoparticle comprises 0-90 at.% (relative to Si) sulfur atoms, including all 0.1 at.% values and ranges therebetween. In certain embodiments, a silica or aluminosilicate nanoparticle comprises 5-90 at.% (relative to Si), 5-60 at.% (relative to Si), 5-80 at.% (relative to Si), 10-80 at.% (relative to Si), or 30-60 at.% (relative to Si) sulfur atoms.

In certain embodiments, a silica or aluminosilicate nanoparticle comprises 0-90 at.% (relative to Si) (e.g., 10-80 and 30-60 at.% (relative to Si)). The sulfur atom(s) are covalently bonded to the silica network of the silica nanoparticle or covalently bonded to the aluminosilicate network of the aluminosilicate nanoparticle, at least one dye molecule (e.g., 1, 2, 3, 4, or 5 dye molecules) covalently bonded thereto, and a longest dimension of less than 10 nm (e.g., 0.01-9.99 nm, including all 0.01 nm values and ranges therebetween).

In certain embodiments, a silica nanoparticle or aluminosilicate nanoparticle comprises one or more heavy atoms. In the case where the heavy atom is iodine, such nanoparticles are also referred to herein as iC' dots. The heavy atoms may either be covalently bonded to the silica network of the silica nanoparticle or the aluminosilicate network of the aluminosilicate nanoparticle or covalently bound to a surface of the nanoparticle or non-covalently bound (e.g, chelated) to a surface of the nanoparticle. A nanoparticle can comprise a mixture of two or more different heavy atoms. The heavy atoms may be either covalently bonded the silica network of the silica nanoparticle or the aluminosilicate network of the aluminosilicate nanoparticle or covalently bound to a surface of the nanoparticle or non-covalently bound (e.g., chelated) to the silica or aluminosilica network. A nanoparticle can comprise a mixture of two or more different heavy atoms.

Heavy atoms can be incorporated into a silica or aluminosilicate nanoparticle by using a heavy atom-containing precursor in the synthesis of the nanoparticle or non-covalently bound (e.g., chelated) to pre-formed nanoparticle. A heavy atom may be a neutral atom or a metal ion. Non-limiting examples of neutral heavy atoms include iodine atom, bromine atom, and the like covalently bonded to the silica network of the silica nanoparticle or the aluminosilicate network of the aluminosilicate nanoparticle or covalently bound to a surface of the nanoparticle. Non-limiting examples of metal ions include Au ions, Ag ions, Pb ions, Ti ions, Bi ions, Pt ions, In ions, Sn ions, Sb ions or Pd ions, and the like non-covalently bound (e.g., chelated) to a portion of a surface of the nanoparticle and/or non-covalently bound (e.g., chelated) to the silica network of a silica nanoparticle or aluninosilica network of an aluminosilicate nanoparticle.

A nanoparticle can comprise various amounts of heavy atom(s). For example, a silica or aluminosilicate nanoparticle comprises 0-20 at.% (relative to Si) heavy atoms, including all 0.1 at% values and ranges therebetween. In certain embodiments, a silica or aluminosilicate nanoparticle comprises (e.g., 1-20 at.% (relative to Si) and 1-10 at.% (relative to Si)) heavy atoms.

In certain embodiments, a silica or aluminosilicate nanoparticle comprises 0-80 at.% (relative to Si) heavy atoms (e.g., 0-70, 0-60, 1-30, or 1-10 at.% (relative to Si)), at least one fluorescent dye molecule (e.g., 1 or 2, dye molecules) covalently bonded thereto, and a longest dimension of less than 10 nm (e.g., 1-9.99 nm, including all 0.01 nm values and ranges therebetween).

In certain embodiments, the nanoparticle is spherical. In certain embodiments, the nanoparticle is non-spherical. In certain embodiments, the nanoparticle is or comprises a material selected from the group consisting of metal/semi-metal/non-metals, metal/semi-metal/non-metal-oxides, -sulfides, -carbides, -nitrides, liposomes, semiconductors, and/or combinations thereof. In certain embodiments, the metal is selected from the group consisting of gold, silver, copper, and/or combinations thereof.

The nanoparticle may comprise metal/semi-metal/non-metal oxides including silica ($SiO_2$), titania ($TiO_2$), alumina ($Al_2O_3$), zirconia ($Z_rO_2$), germania ($GeO_2$), tantalum pentoxide ($Ta_2O_5$), $NbO_2$, etc., and/or non-oxides including metal/semi-metal/non-metal borides, carbides, sulfide and nitrides, such as titanium and its combinations (Ti, $TiB_2$, TiC, TiN, etc.).

For example, in certain embodiments, an ultra-small (e.g., having a diameter range from 5 nm to 10 nm), was tested in humans as is described in U.S. Publication No. 2014/0248210 A1.

In this example, five patients had no adverse events and the agent was well tolerated over the study period. Pharmacokinetic behavior, expressed as the percentage of the injected dose per gram of tissue (%ID/g), versus time post-injection and the corresponding mean organ absorbed doses, were comparable to those found for other commonly used diagnostic radiotracers. Serial PET imaging of this representative patient showed progressive loss of presumed blood pool activity from major organs and tissues, with no appreciable activity seen by 72-hour post-injection (p.i.). Whole-body clearance half-times in these patients were estimated to range from 13-21 hours. Interestingly, there was no notable localization in the liver, spleen, or bone marrow, in contrast to many hydrophobic molecules, proteins, and larger particle platforms (greater than 10 nm). Although patients were pretreated with potassium iodide (KI) to block thyroid tissue uptake, a higher average absorbed thyroid dose was obtained in this patient relative to other tissues. Particles were also primarily excreted by the kidneys, with both kidney and bladder wall (after thyroid and tumor), demonstrating one of the highest %ID/g values by 72 hrs p.i.; as is often the case for renally excreted radiopharmaceuticals, the bladder wall received a higher average absorbed dose than other major organs and tissues. These findings highlight the fact that renal, rather than hepatobiliary, excretion is the predominant route of clearance from the body.

The nanoparticle comprises one or more polymers, e.g., one or more polymers that have been approved for use in humans by the U.S. Food and Drug Administration (FDA) under 21 C.F.R. § 177.2600, including, but not limited to, polyesters (e.g., polylactic acid, poly(lactic-co-glycolic acid), polycaprolactone, polyvalerolactone, poly(1,3-dioxan-2-one)); polyanhydrides (e.g., poly(sebacic anhydride)); polyethers (e.g., polyethylene glycol); polyurethanes; polymethacrylates; polyacrylates; polycyanoacrylates; copolymers of PEG and poly(ethylene oxide) (PEO).

The nanoparticle may comprise one or more degradable polymers, for example, certain polyesters, polyanhydrides, polyorthoesters, polyphosphazenes, polyphosphoesters, certain polyhydroxyacids, polypropylfumerates, polycaprolactones, polyamides, poly(amino acids), polyacetals, polyethers, biodegradable polycyanoacrylates, biodegradable polyurethanes and polysaccharides. For example, specific biodegradable polymers that may be used include but are not limited to polylysine, poly(lactic acid) (PLA), poly(glycolic acid) (PGA), poly(caprolactone) (PCL), poly(lactide-co-glycolide) (PLG), poly(lactide-co-caprolactone) (PLC), and poly(glycolide-co-caprolactone) (PGC). Another exemplary degradable polymer is poly (beta-amino esters), which may be suitable for use in accordance with the present application.

In certain embodiments, a nanoparticle can have or be modified to have one or more functional groups. Such functional groups (within or on the surface of a nanoparticle) can be used for association with any agents (e.g., detectable entities, targeting entities, therapeutic entities, or PEG). In addition to changing the surface charge by introducing or modifying surface functionality, the introduction of different functional groups allows the conjugation of linkers (e.g., (cleavable or (bio-) degradable) polymers such as, but not limited to, polyethylene glycol, polypropylene glycol, PLGA, etc.), targeting/homing agents, and/or combinations thereof.

In certain embodiments, the nanoparticle comprises a therapeutic agent, e.g., a drug moiety (e.g., a chemotherapy drug) and/or a therapeutic radioisotope. As used herein, "therapeutic agent" refers to any agent that has a therapeutic effect and/or elicits a desired biological and/or pharmacological effect, when administered to a subject.

For example, the nanoparticles described herein demonstrate enhanced penetration of tumor tissue (e.g., brain tumor tissue) and diffusion within the tumor interstitium, e.g., for treatment of cancer (e.g., gliomas, e.g., high grade gliomas), as described in PCT/US17/30056 ("Compositions and Methods for Targeted Particle Penetration, Distribution, and Response in Malignant Brain Tumors," filed April 28, 2016) by Bradbury et A1.

Further described are methods of targeting tumor-associated macrophages, microglia, and/or other cells in a tumor microenvironment using such nanoparticles.

Moreover, diagnostic, therapeutic, and theranostic (diagnostic and therapeutic) platforms featuring such nanoparticle conjugates are described for treating targets in both the tumor and surrounding microenvironment, thereby enhancing efficacy of cancer treatment. Use of the nanoparticles described herein with other conventional therapies, including chemotherapy, radiotherapy, immunotherapy, and the like, is also envisaged.

Multi-targeted kinase inhibitors and combinations of single-targeted kinase inhibitors have been developed to overcome therapeutic resistance. Importantly, multimodality combinations of targeted agents, including particle-based probes designed to carry small molecule inhibitors (SMIs), chemotherapeutics, radiotherapeutic labels, and/or immunotherapies can enhance treatment efficacy and/or improve treatment planning of malignant brain tumors. Coupled with molecular imaging labels, these vehicles permit monitoring of drug delivery, accumulation, and retention, which may, in turn, lead to optimal therapeutic indices.

Moreover, use of radiolabels and/or fluorescent markers attached to (or incorporated in or on, or otherwise associated with) the nanoparticles provide quantitative assessment of particle uptake and monitoring of treatment response. In various embodiments, modular linkers are described for incorporating targeting ligands to develop a drug delivery system with controlled pharmacological properties. The described platforms determine the influence of targeting on nanoparticle penetration and accumulation, thereby establishing an adaptable platform for improved delivery of a range of tractable SMIs, for example, to primary and metastatic brain tumors.

In certain embodiments, the nanoparticle comprises one or more targeting ligands (e.g., attached thereto), such as, but not limited to, small molecules (e.g., folates, dyes, etc.), aptamers (e.g., A10, AS1411), polysaccharides, small biomolecules (e.g., folic acid, galactose, bisphosphonate, biotin), oligonucleotides, and/or proteins (e.g., (poly)peptides (e.g., αMSH, RGD, octreotide, AP peptide, epidermal growth factor, chlorotoxin, transferrin, etc.), antibodies, antibody fragments, proteins, etc.). In certain embodiments, the nanoparticle comprises one or more contrast/imaging agents (e.g., fluorescent dyes, (chelated) radioisotopes (SPECT, PET), MR-active agents, CT-agents), and/or therapeutic agents (e.g., small molecule drugs, therapeutic (poly)peptides, therapeutic antibodies, (chelated) radioisotopes, etc.).

In certain embodiments, PET (Positron Emission Tomography) tracers are used as imaging agents. In certain embodiments, PET tracers comprise $^{89}$Zr, $^{64}$Cu, [$^{18}$F] fluorodeoxyglucose. In certain embodiments, the nanoparticle includes these and/or other radiolabels.

The nanoparticle comprises two photoluminescent dyes and possibly or more fluorophores. Fluorophores comprise fluorochromes, fluorochrome quencher molecules, metal chelates, or any fluorescent enzyme substrates, including protease activatable enzyme substrates. In certain embodiments, fluorophores comprise long chain carbophilic cyanines. In other embodiments, fluorophores comprise DiI, DiR, DiD, and the like. Fluorochromes comprise far red, and near infrared fluorochromes (NIRF). Fluorochromes include but are not limited to a carbocyanine and indocyanine fluorochromes. In certain embodiments, imaging agents comprise commercially available fluorochromes including, but not limited to Cy5.5, Cy5 and Cy7 (GE Healthcare); Alexa-Flour660, AlexaFlour680, AlexaFluor750, and Alexa-Fluor790 (Invitrogen); VivoTag680, VivoTag-S680, and VivoTag-S750 (VisEn Medical); Dy677, Dy682, Dy752 and Dy780 (Dyomics); DyLight547, DyLight647 (Pierce); HiLyte Fluor 647, HiLyte Fluor 680, and HiLyte Fluor 750 (AnaSpec); IRDye 800CW, IRDye 800RS, and IRDye 700DX (Li-Cor); and ADS780WS, ADS830WS, and ADS832WS (American Dye Source) and Kodak X-SIGHT 650, Kodak X-SIGHT 691, Kodak X-SIGHT 751 (Carestream Health).

In certain embodiments, the nanoparticle comprises (e.g., has attached) one or more targeting ligands, e.g., for targeting cancer tissue/cells of interest.

In certain embodiments, the nanoparticles comprise from 1 to 20 discrete targeting moieties (e.g., of the same type or different types), wherein the targeting moieties bind to receptors on tumor cells (wherein the nanoparticles have an average diameter no greater than 10 nm, e.g., from about 5 nm to about 7 nm, e.g., about 6 nm). In certain embodiments, the 1 to 20 targeting moieties comprises alpha-melanocyte-stimulating hormone (αMSH). In certain embodiments, the nanoparticles comprise a targeting moiety (e.g., αMSH).

Example therapeutics and/or drugs that can be used include RTK inhibitors, such as dasatinib and gefitinib, can target either platelet-derived growth factor receptor (PDGFR) or EGFRmt+ expressed by primary tumor cells of human or murine origin (e.g., genetically engineered mouse models of high-grade glioma, neurospheres from human patient brain tumor explants) and/or tumor cell lines of non-neural origin. Dasatinib and gefitinib analogs can be synthesized to enable covalent attachment to several linkers without perturbing the underlying chemical structure defining the active binding site.

Cancers that may be treated include, for example, prostate cancer, breast cancer, testicular cancer, cervical cancer, lung cancer, colon cancer, bone cancer, glioma, glioblastoma, multiple myeloma, sarcoma, small cell carcinoma, melanoma, renal cancer, liver cancer, head and neck cancer, esophageal cancer, thyroid cancer, lymphoma, pancreatic (e.g., BxPC3), lung (e.g., H1650), and/or leukemia.

In certain embodiments, the nanoparticle comprises a therapeutic agent, e.g., a drug moiety (e.g., a chemotherapy drug) and/or a therapeutic radioisotope.

As used herein, "therapeutic agent" refers to any agent that has a therapeutic effect and/or elicits a desired biological and/or pharmacological effect, when administered to a subject.

The surface chemistry, uniformity of coating (where there is a coating), surface charge, composition, concentration, frequency of administration, shape, and/or size of the nanoparticle can be adjusted to produce a desired therapeutic effect.

In certain embodiments, the nanoparticle comprises a chelator, for example, 1,4,8,1l-tetraazabicyclo[6.6.2]hexadecane-4,l 1- diyl)diacetic acid (CB-TE2A); desferoxamine (DFO); diethylenetriaminepentaacetic acid (DTPA); 1,4,7, 10-tetraazacyclotetradecane-1,4,7, 10-tetraacetic acid (DOTA); thylenediaminetetraacetic acid (EDTA); ethylene glycolbis(2-aminoethyl)-N,N,N',N'- tetraacetic acid (EGTA); 1,4,8,1 l-tetraazacyclotetradecane-l,4,8,l l-tetraacetic acid (TETA); ethylenebis-(2-4 hydroxy-phenylglycine) (EHPG); 5-Cl-EHPG; 5Br-EHPG; 5- Me-EHPG; 5t-Bu-EHPG; 5-sec-Bu-EHPG; benzodiethylenetriamine pentaacetic acid (benzo-DTPA); dibenzo-DTPA; phenyl-DTPA, diphenyl-DTPA; benzyl-DTPA; dibenzyl DTPA; bis-2 (hydroxybenzyl)-ethylenediaminediacetic acid (HBED) and derivatives thereof; Ac-DOTA; benzo-DOTA; dibenzo-DOTA; 1,4,7-triazacyclononane N,N',N"- triacetic acid (NOTA); benzo-NOTA; benzo-TETA, benzo-DOTMA, where DOTMA is 1,4,7, 10-tetraazacyclotetradecane-l,4,7,10-tetra(methyl tetraacetic acid), benzo-TETMA, where TETMA is 1,4,8,1 l-tetraazacyclotetradecane-l,4,8,l l-(methyl tetraacetic acid); derivatives of 1,3-propylenediaminetetraacetic acid (PDTA); triethylenetetraaminehexaacetic acid (TTHA); derivatives of 1,5,10-N,N',N"-tris(2,3- dihydroxybenzoyl)-tricatecholate (LICAM); and l,3,5-N,N',N"-tris(2,3-dihydroxybenzoyl)aminomethylbenzene (MECAM), or other metal chelators.

In certain embodiments, the nanoconjugate comprises more than one chelator.

In certain embodiments the radioisotope-chelator pair is $^{89}$Zr-DFO. In certain embodiments the radioisotope-chelator pair is $^{177}$Lu-DOTA. In certain embodiments, the radioisotope-chelator pair is $^{225}$Ac-DOTA.

In certain embodiments, ultrasmall particles may be associated with PET labels and/or optical probes. Nanoparticles may be observed *in vivo* (e.g., via PET) to evaluate drug accumulation in a target site. For example, nanoparticles with PET labels (e.g., without drug substances) may be administered first. Then, by analyzing the *in vivo* PET images of the nanoparticles, drug (e.g., conjugated with nanoparticles) concentration and accumulation rate in the tumor may be estimated. The dose may be determined based on the obtained estimation to provide personalized medicine (e.g., tumor size rather than the patient's body weight). In certain embodiments, a radiolabeled drug may be traced *in vivo.* A highly concentrated chemotherapy drug is potentially dangerous if it is not targeted. In certain embodiments, nanoparticles with optical probes (e.g., fluorophore) may be used for intraoperative imaging (e.g., where surface of tissue/tumor is exposed) and/or biopsies of tumors.

*Imaging systems and methods*

In certain embodiments, nanoparticles may be imaged using the systems, methods, and apparatus as described by Bradbury et al. US Publication No. US 2015/0182118 A1, "Systems, Methods, and Apparatus for Multichannel Imaging of Fluorescent Sources in Real Time".

In certain embodiments, nanoparticles may be imaged using one or more of the following systems: stochastic optical reconstruction microscopy (STORM), ground state depletion (GSD) microscopy, direct stochastic optical reconstruction microscopy (dSTORM), stimulated emission and depletion (STED), and photoactivated localization microscopy (PALM).

In certain embodiments, nanoparticles may be imaged using a functional camera system. A functional camera system can offer the ability to gather both structural (e.g., anatomical) and dynamic information (e.g., perfusion, e.g., hypoxia) from nanoparticles localized within one or more cells of the tissue of the subject (e.g., within one or more cellular compartments and/or structures), and can facilitate decisions made during medical procedures. By contrast, conventional imaging methods require excised tissue, and therefore are limited to structural information only.

## Constructive Examples

### *Self-programmable nanotherapies (e.g., for clinical care)*

The present disclosure is directed to systems and methods for controlled delivery and release of metabolites/nanomedicines to regulate tumor microenvironment, cellular viability. For example, the systems and methods comprise delivery and/or release of pathway inhibitors and/or other immune modulators on the basis of sensing pH, ROS for reprogramming tumor-infiltrating macrophages, effector cells, and/or improving responses to T-cell checkpoint immunotherapy.

### *"On-demand" treatment and monitoring during surgery*

The present disclosure is also directed to systems and methods for continuous, nano-sensor-driven patient monitoring and 'on-demand' treatment in surgical settings. For example, the provided systems and methods allow for continuous, real-time monitoring of surgical/adjuvant treatment responses. Moreover, the systems and methods comprise activating metabolic 'triggers or switches' to adjust for deficient or excess metabolite levels by releasing substrates at the tissue/cellular level or adsorbing excess metabolites, and can serve as, for example, surgical tool for predicting post-operative recovery.

An application that may benefit from the disclosed technology includes compartment syndrome. Compartment syndrome arises when excessive pressure builds up within the muscles following a traumatic injury, for example, that can restrict blood flow to muscles and nerves- a surgical emergency. Nano-sensor-driven surgical patient monitoring can be used to predict post-operative recovery, other sequelae (e.g., compartment syndrome, graft replacement, tissue/organ transplants) on the basis of ROS, pH/perfusion alterations.

## Combined treatment and metabolic sensing

Accordingly, the present disclosure is directed to systems and methods for performing real-time quantitative assays of metabolic derangements and fluxes at subcellular resolution (e.g., organelles vs. cytosol) in cancerous cells and tissues.

For example, the provided systems and methods are used for testing and/or monitoring nanomedicine efficacy. In particular, reactive oxygen species (ROS), lipid peroxidation, pH perturbations, iron levels, calcium, amino acids, for example, are detected to monitor cancer cell state and/or responses to targeted therapies in specific organelle compartments (e.g., lysosome, mitochondria, plasma membrane, nucleus).

The imaging systems and methods may allow for detection of cell death and/or other metabolic changes associated with growth arrest, senescence, cell polarization or differentiation, or other altered cell phenotypes.

Moreover, in certain embodiments, the systems and methods are used for continuous kinetic monitoring of metabolic species within the cell (e.g., oxygen (ROS), pH, amino acids (e.g., leucine, glutamine, arginine), iron, glutathione, etc.) to monitor concentration changes within the cell, and, for example, to provide real-time readouts of metabolic state and treat substrate and/or metabolite derangements in organelles to control/prevent cell growth, division, translation, oncogenic signaling, etc.

The present disclosure describes nanoparticles (e.g., nanosensors, e.g., photoswitchable nanoparticles) with engineered capability to sense changes in cellular or extracellular pH or ROS. The nanoparticles can be utilized to track changes in cancer microenvironment linked to progression (reduced extracellular pH) or therapeutic responses (increased lysosomal pH or decreased cellular pH, or increased cellular or lysosomal ROS). For example, as described in the Examples, pH-detecting nanoparticles can be loaded with two fluorophores (FITC and ATTO647N) and be utilized for ratiometric imaging and/or sensing (green and red fluorescence) to indicate relative pH. pH (or ROS)-detecting nanosensor-treated cancer cells (M21 melanoma) can be imaged by confocal and/or super-resolution microscopy to quantify subcellular or extracellular localization, as well as particle intensities to indicate changes in pH and/or accumulation of ROS.

In certain embodiments, ratiometric imaging and/or sensing can be determined as described by Burns et al. "Photoluminescent Silica-Based Sensors and Methods of Use," in US Patent No. US 8,084,001 B1.

Burns et al. describes that major emission peak intensities of two or more photoluminescent dyes can be measured under known environment or analyte conditions. A calibration can be performed by calculating ratios of peak emission for two dyes (e.g., a sensor dye, e.g., a reference dye); the ratios correspond to known conditions.

Moreover, the calibration forms a basis for determinations of unknown conditions through excitation and emission intensity measurement of nanoparticles comprising the same reference and sensor dyes under the unknown conditions. For example, unknown environmental or analyte conditions are investigated through the use of calibration curves that are established based on the wavelengths of maximal emission exhibited by reference and sensor dyes upon excitation under known conditions. That is, rather than relying upon ratios of peak emission intensities, however, the difference between maximum peak wavelengths ($\lambda_{max}$) for the reference and sensor dyes is used as a dependent variable.

Absolute pH quantifications can be made based on standard curves generated by bathing cells in pH-adjusted buffers. The effects of induction of cell death (e.g., apoptosis, e.g., ferroptosis) on intracellular or lysosomal pH can be determined in culture, prior to studies designed to detect the effects of therapeutic approaches on experimental xenograft tumors, using imaging of thin fixed or frozen sections. For example, lifetime-based sensing measures the excited state lifetimes of two or more photo luminescent dyes, one of which whose lifetime is known to be environmentally insensitive, and at least one of which whose lifetime is dependent upon a specific environmental condition or analyte. The fluorescence lifetime ($\tau$) is the length of time that a photoluminescent dye spends in an excited state, from initial absorption of a photon until its emission; it is most often in the range from 10 ps to several nanoseconds. A calibration may be performed by measurement of the fluorescence lifetimes for multiple analyte concentrations, creating a basis for further concentration determinations.

For example, nanoparticles described herein can be introduced into a cellular environment of unknown pH. The nanoparticles are then excited by one or more selected wavelengths of a multi-photon light source, typically exhibiting an excitation wavelength between about 250.0 nm and about 800.0 nm, as appropriate for the absorption profiles of the reference and sensor dyes in the particle. Multiphoton excitation utilizes lasers (often Ti-Sapphire), which can be tuned to a variety of emission wavelengths. The long wavelength (700.0 nm to >1500.0 nm) photons are not readily absorbed by tissue or media but may induce multiphoton excitation in dye molecules at very high photon flux (e.g., at the focal point of the beam). While the reference dye typically exhibits a relatively constant wavelength and intensity of emission, the sensor dye exhibits emissions that correspond to environmental stimuli or to the presence or concentration of certain analytes. Thus, after excitation of each of the reference and sensor dyes at specific levels, photon emissions may be measured. In certain embodiments, after the comparison data have been generated, they may be used to ascertain unknown environmental conditions or the presence and concentration of analytes. Nanoparticle emissions are recorded in the presence of the unknown condition or analyte, and the data are used to determine the unknown condition by comparing it with the comparison data established for the known conditions.

Therapeutic approaches to be examined in combination with pH sensing can include treatment with ultrasmall nanoparticles (e.g., C'dots), imaged separately in the C5 channel. Without wishing to be bound to any theory, it is noted that this approach can further determine the extent of particle internalization into cancer cells in tumor sections, as extracellular-localized particles are predicted to exhibit markedly increased pH profiles as compared to those within lysosomes. Relative changes in extracellular and intracellular pH in response to treatment can also be assessed.

In vitro studies can serve to inform the application of these pH/ROS sensor particles for monitoring graft and/or tissue viability at sites of surgical intervention/resection in conjunction with a functional camera system in small- / larger animal models. Nanoparticles can be implanted directly within the post-operative bed/graft site or injected intravenously. Continuous optical readouts will provide quantitative information on pH, free radicals (e.g., specific species; global amount of oxidative species), perfusion, and deoxy- /oxyhemoglobin.

*Super-resolution microscopy and photoswitchable nanoparticles*

The present disclosure is also directed to systems and methods for tracking targeted drug and/or metabolite delivery using photoswitchable nanoparticles. For example, photoswitchable nanoparticles are used in applications such as tracking to subcellular compartments in human cancers, and performing 'optical biopsies" and super-resolution nanoscopy during surgery (e.g., using artificial neural networks/machine learning). Moreover, photoswitchable nanoparticles function as metabolic sensors, and monitor a variety of metabolites and substrates.

Examples of photoswitchable nanoparticles include sub-10 nm fluorescent silica nanoparticles as described by Kohle et al., "Sulfur- or Heavy Atom-Containing Nanoparticles, Methods or Making the Same, and Uses Thereof," in International Application No. PCT/US18/26980 filed on April 10, 2018.

For example, Kohle et al. describes molecular engineering of ultrasmall sub-10nm fluorescent silica probes by tuning the fluorescent properties of the silica particles (e.g., by altering the chemical composition of the silica network). For instance, by strategically altering the precise chemical environment around the covalently bound dye inside the silica network, Kohle et al. shows that intrinsic electronic properties of organic dyes leading to different photonic behavior as compared to their counterparts in solution can be influences. This is demonstrated by using (3-Mercaptopropyl)trimethoxysilane (MPTMS) or (3-Iodopropyl)trimethoxysilane (IPTMS) as precursors together with Tetramethyl orthosilicate (TMOS) to form sub 10-nm particles. The relative precursor amount of MPTMS to TMOS can be changed from 0% to 90% and the relative precursor amount of IPTMS to TMOS can be changed from 0% to 70%. Furthermore, the synthesis is based in water, it is easy to perform, particles are stable and can be functionalized. The synthesis is applicable to a wide range of commercially available precursors and dyes. It could be shown that such probes can be used in stochastic optical reconstruction microscopy (STORM) to achieve resolutions below the diffraction limit. Nanoparticles may also be imaged using one or more of the following systems: ground state depletion (GSD) microscopy, direct stochastic optical reconstruction microscopy (dSTORM), stimulated emission and depletion (STED), and photoactivated localization microscopy (PALM).

*App-driven (e.g., iPad) real-time surgical imaging systems*

The present disclosure is also directed to systems and methods for super-resolution, interactive, multifunctional imaging, surgical navigation, and cancer treatment planning. For example, the systems and methods described herein are capable of real time mapping of (i) tissue perfusion, viability, oxygen/pH status, (ii) deep tissues, (iii) tumor volumes for surgical navigation, and (iv) treatment planning volumes for perioperative high-precision therapies. Moreover, the presented technology can, for example, perform treatment planning volumes from pre-operative imaging (CT, etc.) co-registered with augmented reality-generated images projected over the operative bed to permit direct application of tumor margin mapping.

*Mapping of surgical margins*

The present disclosure is also directed to systems and methods for mapping surgical margins with cellular-level precision using super-resolution imaging. For example, the described technology allows for co-registration of *in vivo* and *ex vivo* images with *ex vivo* super-resolution microscopy of tissue section to facilitate 'on the fly' surgical treatment/management decision-making.

*Super-resolution microscopy, and related methods*

The present disclosure is also directed to super-resolution tracking of drug delivery, metabolite delivery, radiotherapy, ferroptotic induction (e.g., by administration of nanoparticles), and the like, using optically-driven technologies (e.g., super-resolution microscopy). Various embodiments for which such tracking may be employed include therapeutic methods, combination therapies, and surgical procedures. For example, it is possible to monitor drug and/or substrate delivery and trafficking to subcellular compartments in human cancers (e.g., in perioperative settings, in situ and/or ex vivo). Drug/substrate delivery can be monitored at the level of specific organelles (e.g., lysosome, mitochondria, plasma membrane, and/or nucleus) and/or the cytosol, for assessment of treatment efficacy and/or fine-tuning treatment responses. Delivery efficiency and/or subcellular localization of the drug, nanoparticle, and/or other administered substrate may be assessed at a given point in time, or may be tracked over time, e.g., over a treatment period.

Nanoparticle delivery into cells largely occurs through endocytic mechanisms, but such localization inside of cells has been investigated only through conventional microscopy techniques with limited spatial resolution (~200nm resolution). Tracking of single nanoparticles (e.g., C dots, e.g., used for ferroptotic induction and/or used as probes) at super-resolution to quantify uptake efficiency and localization had not heretofore been performed to examine sub-organelle localization and potential escape of individual nanoparticles from lysosomes, which could be rate-limiting for particle-based drug delivery.

Thus, in certain embodiments, intracellular nanoparticles are imaged by super-resolution microscopy techniques to quantify, at or near single nanoparticle resolution subcellular and sub-organelle localization (e.g., at a resolution of 200 nm or less (where a lower value indicates higher resolution), e.g., 175 nm or less, e.g., 150 nm or less, e.g., 125 nm or less, e.g., 120 nm or less, e.g., 110 nm or less, e.g., 100 nm or less). Tissue sections from treated cancer specimens can also be examined ex-vivo for probe localization at subcellular and sub-organelle resolution by advanced techniques.

For example, in certain embodiments, dual color super-resolution confocal imaging is performed on cells expressing a fluorescent marker of lysosomes and treated with a fluorescent nanoparticle of interest. The intracellular localization of single probes and small clusters of probes either to lysosomes, for instance, or to the surrounding cytosol, are determined and the percentages of co-localization patterns quantified. Cells treated over a time-course and with increasing concentrations are examined. If probes are observed outside of lysosomal membranes, further colocalization studies with additional organelle markers (i.e. mitochondria, endoplasmic reticulum, autophagosomes, etc) are performed to identify additional sites of localization. These analyses can serve as a baseline for continued analysis utilizing probes bearing a range of surface chemistries and/or altered physicochemical properties. Tumor tissue section studies can be performed, for example, using thin Lamp1-GFP xenografted tumor sections (i.e. 2-10um) from mice previously treated with the probe of interest, and similar super-resolution microscopy studies performed to examine intralysosomal versus cytosolic localization of intracellular probes. For these studies, a series of frozen or fixed sections can be examined to define a protocol suitable for super-resolution microscopy.

In certain embodiments, a super-resolution microscope is employed. For example, a STORM/TIRF system (e.g., Nikon) with widefield-FLIM and TIRF-FLIM may be employed (FLIM = Fluorescence Lifetime Imaging). Various laser lines may be used for STORM (2D or 3D capabilities) and TIRF (e.g., 405, 488, 561, and 647 nm laser lines). A Lambert Instruments frequency domain LIFA module for FLIM can be used, either in widefield (LED) or laser (TIRF) mode (e.g., 445 and 514 nm lasers). A Photonics Instruments Micropoint laser may be used for photoablation, bleaching, and/or activation. DG5 may be used for widefield illumination. The system may also include a piezo x,y,z stage, an Andor DU-897 EMCCD camera, an Andor Neo sCMOS camera, a Tokai Hit environmental chamber, various objectives suitable for widefield and/or TIRF microscopy, and acquisition software (e.g., Elements acquisition software, Nikon).

Another example super-resolution microscope that may be employed includes an OMX Blaze 3D-SIM super-resolution microscope (Applied Precision). The microscope system may have, for example, 405, 445, 488, 514, and/or 568 nm lasers for 3D-SIM super-resolution imaging. The system may include a multi-line (e.g., 6-line) SSI module for ultra-rapid conventional imaging (e.g., to supplement super-resolution imaging). The system may further include, for example, a 100x/1.40 NA UPLSAPO oil objective (Olympus); multiple Evolve EMCCD cameras (Photometrics) for simultaneous or sequential acquisition (e.g., three cameras); and/or a heating chamber for live cell imaging.

In addition to localization, tracking, and delivery of nanoparticles, drugs, and/or other administered substances at the sub-cellular, it is possible to simultaneously (or alternatively) monitor pH and/or metabolic species (e.g., oxygen (ROS) and/or glutathione) within cells, e.g., for real-time assessment of therapeutic efficacy. Nano-based sensors may be used to track cancer regression or recurrence in response to treatment. It is possible to detect changes in the cancer microenvironment that are linked to progression (e.g., decreased extracellular pH) or therapeutic response (e.g., increased lysosomal pH, decreased cellular pH, and/or increased cellular/lysosomal ROS). pH (or metabolite)-detecting sensor-treated cancer cells can be imaged by confocal or super-resolution microscopy to quantify subcellular localization, as well as intensities to indicate changes in pH and/or accumulation of specific metabolites. Absolute pH quantifications can be made based on standard curves generated by bathing cells in pH-adjusted buffers. The effects of induction of cell death (e.g. apoptosis) on intracellular or lysosomal pH can be determined in culture, prior to studies designed to detect the effects of therapeutic approaches on experimental xenografted tumors using imaging of thin fixed or frozen sections. Therapeutic approaches can be examined in combination with pH sensing. In certain embodiments, the extent of probe internalization within cancer cells is determined in tumor sections, as extracellular-localized probes are predicted to exhibit markedly increased pH profiles as compared to those within lysosomes. Relative changes in extracellular and intracellular pH in response to a variety of treatments can also be determined. In vitro studies can serve to inform the application of sensor technologies in vivo. A functional camera system can provide complementary real-time assessments of pH, oxygenation status, and small-vessel perfusion. The ability to utilize a range of wavelengths spanning ~400-1000 nm allows for the measurement of different spectral absorption and emission profiles defining proteins, metabolic species, or the optical properties of externally administered probes. Hemoglobin, for instance, has different spectral characteristics in the NIR than deoxyhemoglobin. The functional camera system can discriminate these spectral differences over very discrete bandwidths, allowing spatial spectrometry to be performed. This set-up can also be applied to address key biological questions for a variety of tissue types and chromophores.

For example, in certain embodiments, metabolic imaging of cancer progression and therapy is performed by employing nanosensor delivery. Drug treatment responses and cancer progression are imaged by delivery of nanoparticles with sensor capability for pH and reactive oxygen species (ROS). Particle-intrinsic and drug conjugation-based therapeutics can be imaged in real time for assessment of efficacy, and nano-based sensors may be used to track cancer regression or recurrence in response to treatment. Particles with an engineered capability to sense changes in cellular pH or ROS can be used to track changes in the cancer microenvironment.

In certain embodiments, dual color super-resolution confocal imaging can be performed on cells (e.g., M21 melanoma cells) expressing a fluorescent marker of lysosomes (Lamp1-GFP) and treated with Cy5-fluorescent C' dot nanoparticles. The intracellular localization of single nanoparticles and small nanoparticle clusters either to the lysosome lumen or to the cytosol outside of the lysosomal membrane, can be determined and the percentages of co-localization patterns quantified.

Cells treated over a time-course and with increasing concentrations can be examined, including up to 15 μM, a concentration that causes significant intrinsic cell death-inducing capacity of C' dots linked to the lysosomal delivery of iron (for example, as described by Bradbury et al., International (PCT) Patent Application No. PCT/US2016/034351, "Methods of Treatment Using Ultrasmall Nanoparticles to Induce Cell Death of Nutrient-Deprived Cancer Cells Via Ferroptosis".

If C' dots are observed outside of lysosomal membranes, further colocalization studies with additional organelle markers (e.g., mitochondria, endoplasmic reticulum, autophagosomes) can be performed to identify potential additional specific subcellular localizations in addition to lysosomes. These studies can also form the baseline for continued studies utilizing modified C' dots (with different conjugated ligands or changes in surface or internal chemistry or size) or other particles to examine if particular modifications influence lysosomal accumulation or escape to the cytosol.

For tissue section studies, thin tumor sections (e.g., from about 2 to about 10 μm) can be generated from experimental Lamp1-GFP-expressing M21 xenograft tumors harvested from C'dot-treated mice, and similar super-resolution microscopy studies can be performed to examine intralysosmal versus cytosolic localization of intracellular particles. Frozen tumor sections have been imaged by epifluorescence to localize C' dots inside of cancer cells; for these studies series of frozen or fixed sections can be examined to define a protocol suitable for super-resolution microscopy.

## Therapeutic methods involving ferroptotic induction

A ferroptotic induction step may be used in combination with immunotherapy and/or small molecule drugs to overcome chemo/immuno resistance mechanisms observed in current treatment therapies. Ferroptosis induction has been found to involve the spreading of cell death through cancer cell populations in a wave-like manner, whereby death spreads from treated to untreated cells. In certain embodiments, this propagating feature of ferroptotic cell death may offer high therapeutic potential for the treatment of cancer, as cell death induction could spread in even a small population of cancer cells to achieve a more complete kill (including cancer stem-like cells), than the induction of other death forms (i.e., apoptosis). As ferroptosis-inducing agents, "self-therapeutic" nanoparticles may be used as part of a combinatorial treatment paradigm, along with immune checkpoint blocking antibodies and selective inhibitors of myeloid cell targets, for example, to overcome mechanisms of immune resistance in melanoma patients. Furthermore, immune modulators may be delivered and released to regulate the TME, including tumor-associated macrophages (TAMs) and effector cells, and/or improving responses to T-cell checkpoint immunotherapy. Nanoparticles can be used to selectively target pathways known to influence differentiation and survival of macrophages, as well as their activation or polarization state, such as colony stimulating factor-1 (CSF-1). Tumor models sensitive to TME regulation via this pathway can be targeted with CSF-1 small molecule inhibitors, such as BLZ945. Additional targeted inhibitors can be used in combination to disrupt tumor cell signaling via alternative pathways.

Thus, in certain embodiments, a ferroptotic induction step is performed in combination with immunotherapy and/or small molecule drug administration. In certain embodiments, methods and/or compositions described by Bradbury et al., "Methods and Treatment Using Ultrasmall Nanoparticles to Induce Cell Death of Nutrient Deprived Cancer Cells via Ferroptosis," International (PCT) Patent Application No. PCT/US2016/034351, filed on May 26, 2016.

In certain embodiments, Bradbury et al. may be used, for example, in the ferroptotic induction step in the methods described herein. Furthermore, in certain embodiments, ferroptotic induction is assessed and/or monitored using a super-resolution microscope described herein and/or nanoparticles described herein, e.g., to assess mechanisms associated with derangements of the tumor microenvironment (TME), to reprogram the TME, and/or to adjust the therapy. Examples are presented below for prostate cancer and melanoma using C' dots.

*Metastatic melanoma*

Ferroptosis is a mechanism of cell death that involves iron and reactive oxygen species (ROS)-dependent execution. It is discovered that C' dot nanoparticles can induce ferroptosis, and that intravenous administration of particles to cancer-bearing mice inhibits tumor growth and leads to tumor regression in a ferroptosis-dependent manner (See attached figure below). It has also been discovered that this form of cell death propagates through cancer cell populations, spreading in a wave-like manner, underscoring its potential for cancer therapy as an efficient killing mechanism. This anti-cancer effect has been demonstrated in mouse models of fibrosarcoma and renal carcinoma, and against melanoma, lung, breast, and pancreatic carcinoma cells. It has further been discovered that the administration of zirconium-labeled melanoma-targeted C' dots (bearing MC1-R surface-coated peptides) leads to the efficient elimination of melanoma cells through ferroptosis (see figure below). Together these data demonstrate the utility of C' dot-mediated ferroptosis induction for anti-cancer therapy. Thus, in certain embodiments, ferroptosis-inducing nanoparticles (e.g., ultrasmall silica nanoparticles, e.g., C' dots) are administered as part of a combination therapy with one or more standard-of-care ICB antibodies, along with one or more small molecule inhibitors (or other therapies) targeting myeloid cell populations.

Recent clinical trials using immunotherapy have demonstrated its potential to control cancer by disinhibiting the immune system. Immune checkpoint blocking (ICB) antibodies against cytotoxic-T-lymphocyte-associated protein 4 or programmed cell death protein 1/programmed death-ligand 1 have displayed durable clinical responses in various cancers. Although these new immunotherapies have had a notable effect on cancer treatment, multiple mechanisms of immune resistance exist in tumors. Among the key mechanisms, myeloid cells have a major role in limiting effective tumor immunity. Growing evidence suggests that high infiltration of immune-suppressive myeloid cells correlates with poor prognosis and immunoresistance. These observations suggest a need for a precision medicine approach in which the design of the therapeutic combination is modified on the basis of the tumor immune landscape to overcome such resistance mechanisms. It has also been shown that resistance to ICB is directly mediated by the suppressive activity of infiltrating myeloid cells in various tumors. Selective pharmacologic targeting of the gamma isoform of phosphoinositide 3-kinase (PI3K$\gamma$), highly expressed in myeloid cells, can restore sensitivity to ICB. Targeting PI3K$\gamma$ with a selective inhibitor, is currently being evaluated in a phase 1 clinical trial (NCT02637531); this can reshape the tumor immune microenvironment and promote cytotoxic-T-cell-mediated tumor regression without targeting cancer cells directly. Moreover, to overcome resistance, a unique approach is presented: self-therapeutic nanoparticles (C'-dots) inducing an iron-/ROS-driven cell death program, ferroptosis. Without wishing to be bound by any particular theory, it is hypothesized that wave-like cell-to-cell communication of death-inducing signals promote more efficient tumor necrosis, supported by cancer regression/growth inhibition in-vivo after high-dose i.v.-injected cancer-targeting C'-dots. Melanocortin-1 receptor (MC1-R)-targeted probes, used with cytotoxics/radiopharmaceuticals, internalizes on cell-binding. In certain embodiments, this ferroptosis-inducing capability is exploited with MC1R-targeted C-dots. Efficacy of MC1R-targeted C-dots can be compared as monotherapy or as a combination strategy with ICB antibodies/PI3-Ky inhibitors in genetically-engineered mouse models of melanoma (i.e., B16GM).

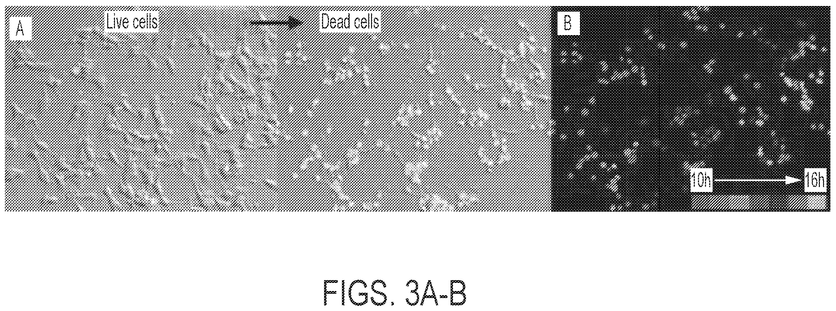

FIGS. 3A-3B show images of melanoma-targeted C'dots that efficiently kill B16 melanoma cells, according ab embodiment described by the present disclosure. FIGS. 3A and 3B show an image depicting that treatment of B16 mouse melanoma cells with $\alpha$MSH-C' dots leads to ferroptosis (middle panel, dead cells indicated with Sytox green staining) that propagates and kills the cell population. Cell nuclei are pseudocolored to indicate the timing of cell death.

Metastatic castration-resistant prostate cancer

Ferroptosis is a recently discovered mechanism of cell death that involves iron and reactive oxygen species (ROS)-dependent execution. It is discovered that C' dot nanoparticles can induce ferroptosis, and that intravenous administration of particles to cancer-bearing mice inhibits tumor growth and leads to tumor regression in a ferroptosis-dependent manner (See figure below). It is also discovered that this form of cell death propagates through cancer cell populations, spreading in a wave-like manner, underscoring its potential for cancer therapy as an efficient killing mechanism. This anti-cancer effect is demonstrated in mouse models of fibrosarcoma and renal carcinoma, and against melanoma, lung, breast, and pancreatic carcinoma cells. It is further discovered that the administration of zirconium-labeled prostate cancer-targeted C' dots (bearing PSMA surface-coated peptides) leads to the efficient elimination of prostate carcinoma cells through ferroptosis when combined with treatment with the standard-of-care anti-androgen receptor therapy enzalutamide (see figure below). Together these data demonstrate the potential utility of C' dot-mediated ferroptosis induction for anti-cancer therapy, and establish the premise for investigating C' dot-based therapy against prostate cancers in combination with standard-of-care anti-androgen receptor therapy (enzalutamide), along with other prostate cancer therapies, such as the hypoxia-activated prodrug, TH-302.

Despite recent drug approvals, more effective therapies are needed for metastatic castration-resistant prostate cancer (mCRPC), accounting for 28,000 deaths (2016), along with pain/spinal cord compromise in thousands more. Limitations of life-prolonging therapies include elimination of quiescent PC stem-like cells persisting posttreatment, and evidence shows castration-resistant cells ultimately reactivating, contributing/evolving to lethal CRPC phenotypes. To overcome resistance, a unique approach is presented: self-therapeutic nanoparticles (C'-dots) inducing an iron-/ROS-driven cell death program, ferroptosis. Without wishing to be bound to a particular theory, it is hypothesized that wave-like cell-to-cell communication of death-inducing signals promote more efficient tumor necrosis, supported by cancer regression/growth inhibition in-vivo after high-dose i.v.-injected cancer targeting C'-dots. CRPC-upregulated prostate-specific membrane antigen (PSMA), used with cytotoxics/radiopharmaceuticals, internalizes on cell-binding. In certain embodiments, this ferroptosis-inducing capability is exploited with PSMA-targeted C'-dots. Efficacy of PSMA-targeted C'-dots can be compared as mono therapy or combined with enzalutamide/TH-302 in PC-models.

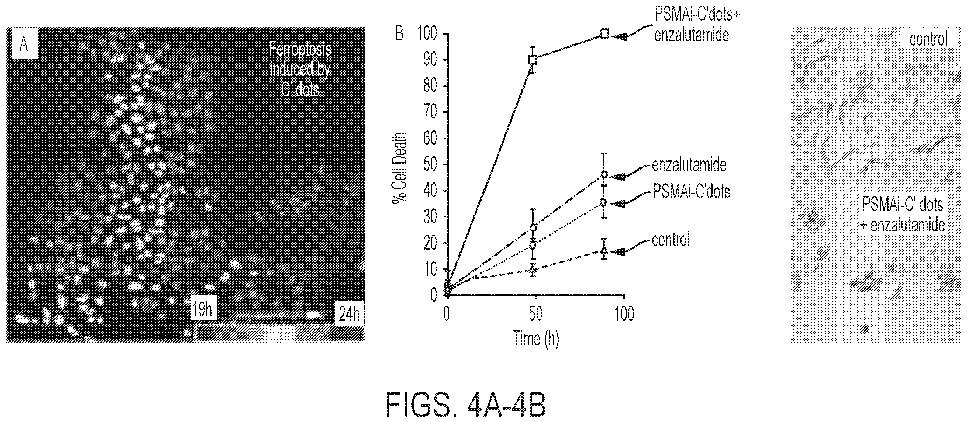

FIGS. 4A and 4B show images and a plot that depict that C' dot nanoparticles induce ferroptosis that spreads through cell populations and kills prostate cancer in combination with enzalutamide. FIG. 4A shows an image depicting that C'dot treated cells undergo ferroptotic cell death that spreads through entire populations in a wave-length manner. Image shows nuclei of dead cells, pseudocolored to indicate the timing of cell death after treatment (from 19-24 hours). FIG. 4B shows a plot that depicts that prostate cancer-targeting PSMAs-C' dots kill androgen-dependent prostate cancer cells (LNCaP) efficiently when combined with enzalutamide. Images show representative control and PSMAi dot and enzalutamide treated LNCaP cells.

*Exemplary network environment*

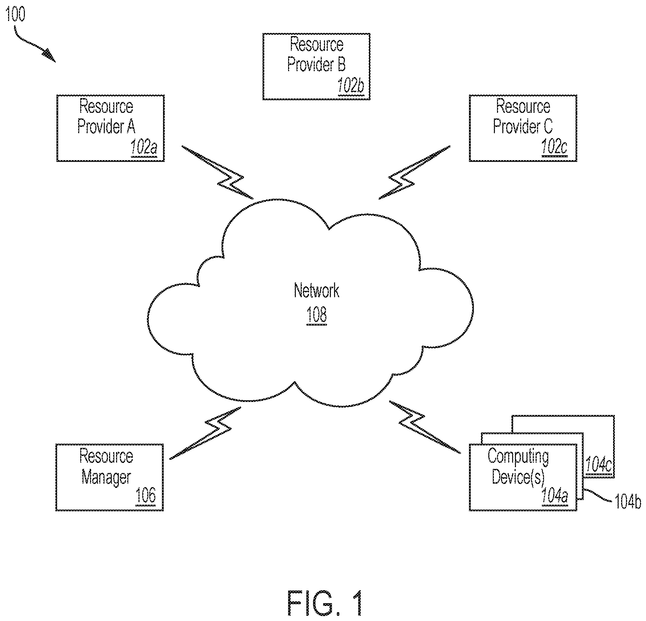

FIG. 1 shows an illustrative network environment 100 for use in the methods and systems for analysis of spectrometry data corresponding to particles of a sample, as described herein. In brief overview, referring now to FIG. 1, a block diagram of an exemplary cloud computing environment 100 is shown and described. The cloud computing environment 100 may include one or more resource providers 102a, 102b, 102c (collectively, 102). Each resource provider 102 may include computing resources. In some implementations, computing resources may include any hardware and/or software used to process data. For example, computing resources may include hardware and/or software capable of executing algorithms, computer programs, and/or computer applications. In some implementations, exemplary computing resources may include application servers and/or databases with storage and retrieval capabilities. Each resource provider 102 may be connected to any other resource provider 102 in the cloud computing environment 100. In some implementations, the resource providers 102 may be connected over a computer network 108. Each resource provider 102 may be connected to one or more computing device 104a, 104b, 104c (collectively, 104), over the computer network 108.

The cloud computing environment 100 may include a resource manager 106. The resource manager 106 may be connected to the resource providers 102 and the computing devices 104 over the computer network 108. In some implementations, the resource manager 106 may facilitate the provision of computing resources by one or more resource providers 102 to one or more computing devices 104. The resource manager 106 may receive a request for a computing resource from a particular computing device 104. The resource manager 106 may identify one or more resource providers 102 capable of providing the computing resource requested by the computing device 104. The resource manager 106 may select a resource provider 102 to provide the computing resource. The resource manager 106 may facilitate a connection between the resource provider 102 and a particular computing device 104. In some implementations, the resource manager 106 may establish a connection between a particular resource provider 102 and a particular computing device 104. In some implementations, the resource manager 106 may redirect a particular computing device 104 to a particular resource provider 102 with the requested computing resource.

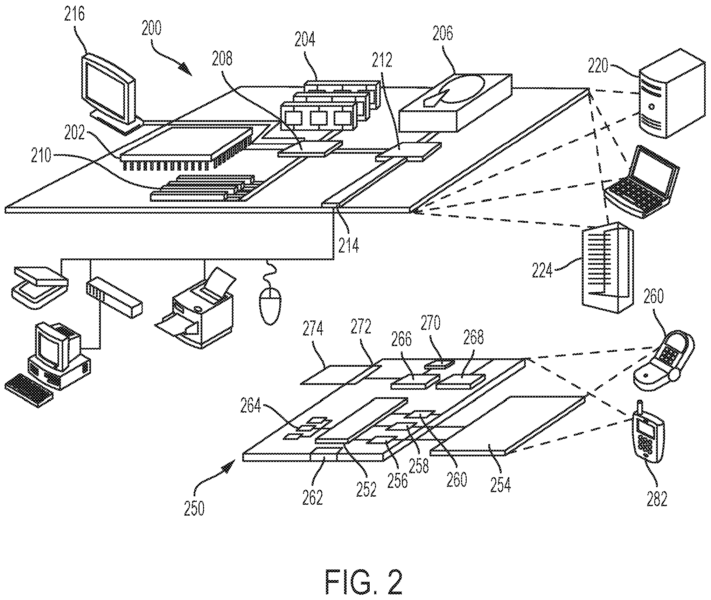

FIG. 2 shows an example of a computing device 200 and a mobile computing device 250 that can be used in the methods and systems described in this disclosure. The computing device 200 is intended to represent various forms of digital computers, such as laptops, desktops, workstations, personal digital assistants, servers, blade servers, mainframes, and other appropriate computers. The mobile computing device 250 is intended to represent various forms of mobile devices, such as personal digital assistants, cellular telephones, smartphones, and other similar computing devices. The components shown here, their connections and relationships, and their functions, are meant to be examples only, and are not meant to be limiting.

The computing device 200 includes a processor 202, a memory 204, a storage device 206, a high-speed interface 208 connecting to the memory 204 and multiple high-speed expansion ports 210, and a low-speed interface 212 connecting to a low-speed expansion port 214 and the storage device 206. Each of the processor 202, the memory 204, the storage device 206, the high-speed interface 208, the high-speed expansion ports 210, and the low-speed interface 212, are interconnected using various busses, and may be mounted on a common motherboard or in other manners as appropriate. The processor 202 can process instructions for execution within the computing device 200, including instructions stored in the memory 204 or on the storage device 206 to display graphical information for a GUI on an external input/output device, such as a display 216 coupled to the high-speed interface 208. In other implementations, multiple processors and/or multiple buses may be used, as appropriate, along with multiple memories and types of memory. Also, multiple computing devices may be connected, with each device providing portions of the necessary operations (e.g., as a server bank, a group of blade servers, or a multi-processor system).

The memory 204 stores information within the computing device 200. In some implementations, the memory 204 is a volatile memory unit or units. In some implementations, the memory 204 is a non-volatile memory unit or units. The memory 204 may also be another form of computer-readable medium, such as a magnetic or optical disk.

The storage device 206 is capable of providing mass storage for the computing device 200. In some implementations, the storage device 206 may be or contain a computer-readable medium, such as a floppy disk device, a hard disk device, an optical disk device, or a tape device, a flash memory or other similar solid state memory device, or an array of devices, including devices in a storage area network or other configurations. Instructions can be stored in an information carrier. The instructions, when executed by one or more processing devices (for example, processor 202), perform one or more methods, such as those described above. The instructions can also be stored by one or more storage devices such as computer- or machine-readable mediums (for example, the memory 204, the storage device 206, or memory on the processor 202).

The high-speed interface 208 manages bandwidth-intensive operations for the computing device 200, while the low-speed interface 212 manages lower bandwidth-intensive operations. Such allocation of functions is an example only. In some implementations, the high-speed interface 208 is coupled to the memory 204, the display 216 (e.g., through a graphics processor or accelerator), and to the high-speed expansion ports 210, which may accept various expansion cards (not shown). In the implementation, the low-speed interface 212 is coupled to the storage device 206 and the low-speed expansion port 214. The low-speed expansion port 214, which may include various communication ports (e.g., USB, Bluetooth®, Ethernet, wireless Ethernet) may be coupled to one or more input/output devices, such as a keyboard, a pointing device, a scanner, or a networking device such as a switch or router, e.g., through a network adapter.

The computing device 200 may be implemented in a number of different forms, as shown in the figure. For example, it may be implemented as a standard server 220, or multiple times in a group of such servers. In addition, it may be implemented in a personal computer such as a laptop computer 222. It may also be implemented as part of a rack server system 224. Alternatively, components from the computing device 200 may be combined with other components in a mobile device (not shown), such as a mobile computing device 250. Each of such devices may contain one or more of the computing device 200 and the mobile computing device 250, and an entire system may be made up of multiple computing devices communicating with each other.

The mobile computing device 250 includes a processor 252, a memory 264, an input/output device such as a display 254, a communication interface 266, and a transceiver 268, among other components. The mobile computing device 250 may also be provided with a storage device, such as a micro-drive or other device, to provide additional storage. Each of the processor 252, the memory 264, the display 254, the communication interface 266, and the transceiver 268, are interconnected using various buses, and several of the components may be mounted on a common motherboard or in other manners as appropriate.

The processor 252 can execute instructions within the mobile computing device 250, including instructions stored in the memory 264. The processor 252 may be implemented as a chipset of chips that include separate and multiple analog and digital processors. The processor 252 may provide, for example, for coordination of the other components of the mobile computing device 250, such as control of user interfaces, applications run by the mobile computing device 250, and wireless communication by the mobile computing device 250.

The processor 252 may communicate with a user through a control interface 258 and a display interface 256 coupled to the display 254. The display 254 may be, for example, a TFT (Thin-Film-Transistor Liquid Crystal Display) display or an OLED (Organic Light Emitting Diode) display, or other appropriate display technology. The display interface 256 may comprise appropriate circuitry for driving the display 254 to present graphical and other information to a user. The control interface 258 may receive commands from a user and convert them for submission to the processor 252. In addition, an external interface 262 may provide communication with the processor 252, so as to enable near area communication of the mobile computing device 250 with other devices. The external interface 262 may provide, for example, for wired communication in some implementations, or for wireless communication in other implementations, and multiple interfaces may also be used.

The memory 264 stores information within the mobile computing device 250. The memory 264 can be implemented as one or more of a computer-readable medium or media, a volatile memory unit or units, or a non-volatile memory unit or units. An expansion memory 274 may also be provided and connected to the mobile computing device 250 through an expansion interface 272, which may include, for example, a SIMM (Single In Line Memory Module) card interface. The expansion memory 274 may provide extra storage space for the mobile computing device 250, or may also store applications or other information for the mobile computing device 250. Specifically, the expansion memory 274 may include instructions to carry out or supplement the processes described above, and may include secure information also. Thus, for example, the expansion memory 274 may be provided as a security module for the mobile computing device 250, and may be programmed with instructions that permit secure use of the mobile computing device 250. In addition, secure applications may be provided via the SIMM cards, along with additional information, such as placing identifying information on the SIMM card in a non-hackable manner.

The memory may include, for example, flash memory and/or NVRAM memory (non-volatile random access memory), as discussed below. In some implementations, instructions are stored in an information carrier and, when executed by one or more processing devices (for example, processor 252), perform one or more methods, such as those described above. The instructions can also be stored by one or more storage devices, such as one or more computer- or machine-readable mediums (for example, the memory 264, the expansion memory 274, or memory on the processor 252). In some implementations, the instructions can be received in a propagated signal, for example, over the transceiver 268 or the external interface 262.

The mobile computing device 250 may communicate wirelessly through the communication interface 266, which may include digital signal processing circuitry where necessary. The communication interface 266 may provide for communications under various modes or protocols, such as GSM voice calls (Global System for Mobile communications), SMS (Short Message Service), EMS (Enhanced Messaging Service), or MMS messaging (Multimedia Messaging Service), CDMA (code division multiple access), TDMA (time division multiple access), PDC (Personal Digital Cellular), WCDMA (Wideband Code Division Multiple Access), CDMA2000, or GPRS (General Packet Radio Service), among others. Such communication may occur, for example, through the transceiver 268 using a radio-frequency. In addition, short-range communication may occur, such as using a Bluetooth®, Wi-Fi™, or other such transceiver (not shown). In addition, a GPS (Global Positioning System) receiver module 270 may provide additional navigation- and location-related wireless data to the mobile computing device 250, which may be used as appropriate by applications running on the mobile computing device 250.

The mobile computing device 250 may also communicate audibly using an audio codec 260, which may receive spoken information from a user and convert it to usable digital information. The audio codec 260 may likewise generate audible sound for a user, such as through a speaker, e.g., in a handset of the mobile computing device 250. Such sound may include sound from voice telephone calls, may include recorded sound (e.g., voice messages, music files, etc.) and may also include sound generated by applications operating on the mobile computing device 250.

The mobile computing device 250 may be implemented in a number of different forms, as shown in the figure. For example, it may be implemented as a cellular telephone 280. It may also be implemented as part of a smart-phone 282, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms machine-readable medium and computer-readable medium refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term machine-readable signal refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network (LAN), a wide area network (WAN), and the Internet.

The computing system can include clients and servers. A client and server are generally remote from each other and typically interact through a communication network. The relationship of client and server arises by virtue of computer programs running on the respective computers and having a client-server relationship to each other.

## Experimental Examples

### *Super-resolution microscopy: Counting cRGDY-PEG-C dots localized within lysosomes*

Super-resolution images are needed to localize administered nanoparticles within subcellular compartments, and/or the blood brain barrier.

In this Example, a confocal super-resolution microscope was used to localize ultrasmall silica-based nanoparticles (cRGDY-PEG-C' dots) within subcellular compartments of a cell.

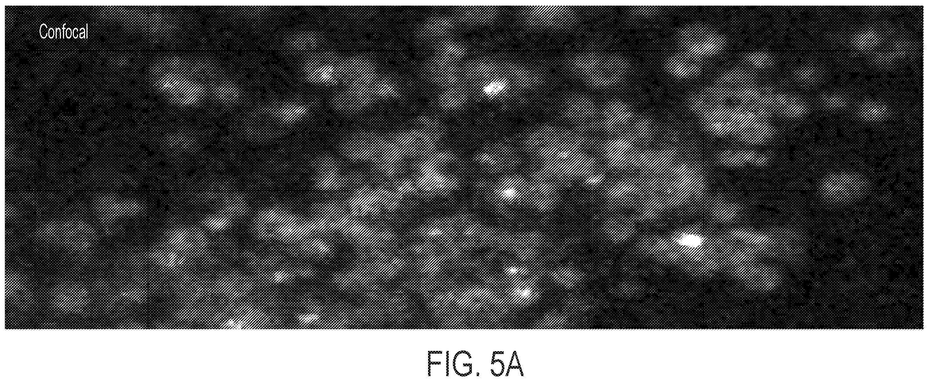

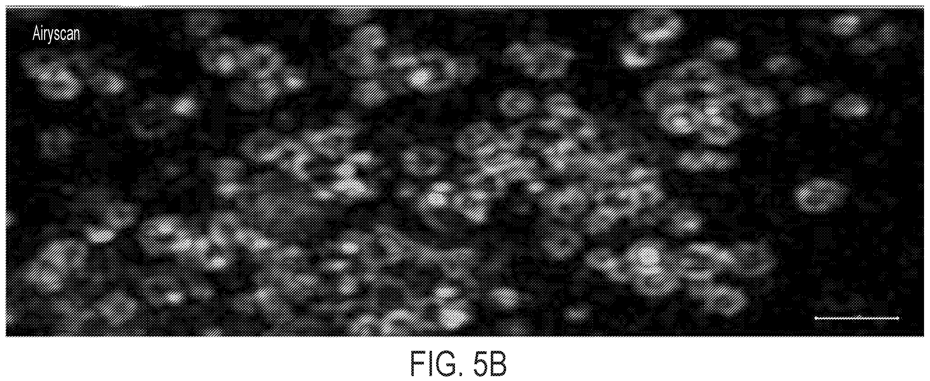

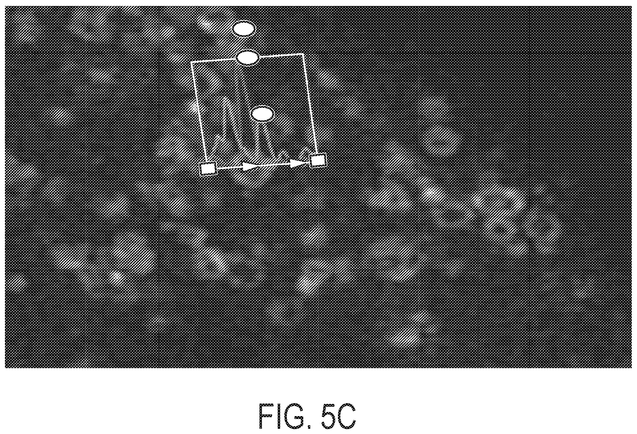

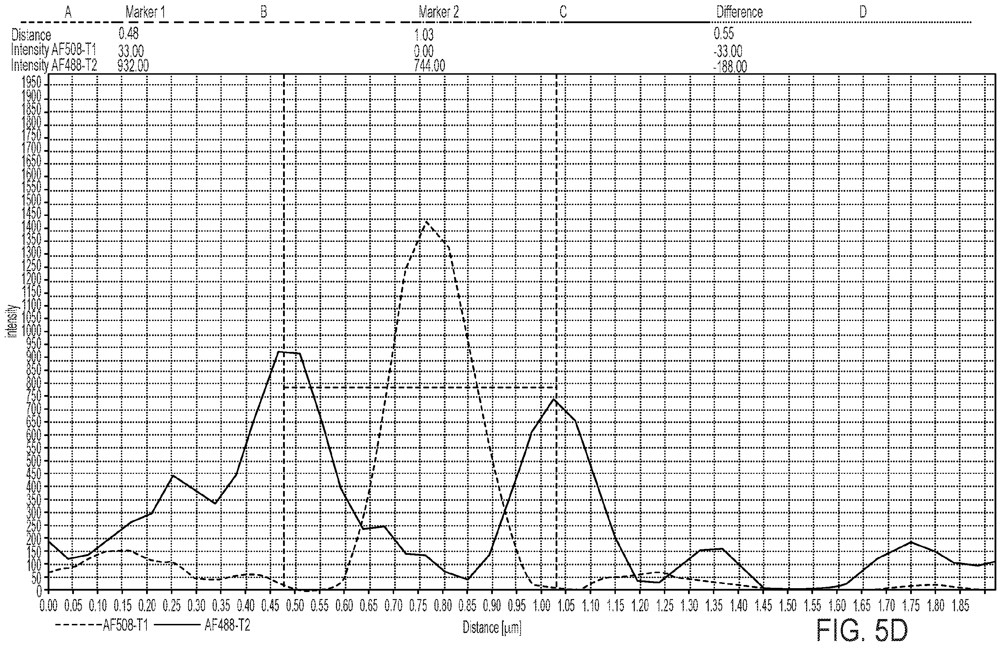

FIGS. 5A-5D show images (FIGS. 5A-5C) and a plot (FIG. 5D) depicting super-resolution microscopy of cRGDY-PEG-C dot lysosomal uptake. PC-3 prostate cancer cells expressing LAMP1-GFP (lysosomal marker, green) were treated with 500 nM cRGDY-PEG-C' dot (red) for 18 hrs. FIG. 5A shows a standard confocal microscopy image using Zeiss LSM-800 laser scanning microscope. FIG. 5B shows an identical imaging field as seen in FIG. 5A, imaged using supper-resolution Airyscan detector (Zeiss). FIGS. 5C-5D show a line profile of isolated lysosome (Green Line) containing cRGDY-PEG-C dots (red line) within the lumen. Scale bar = 1 $\mu$m.

### *Nanosensors for the quantification of intracellular pH*

Prior to the present disclosure, it was unclear whether nanosensors can monitor changes in environmental conditions in diseased cells, such as cancerous cells. This example demonstrates that the present disclosure describes nanosensors that provide quantitative information about the microenvironment. Moreover, the small size of the nanosensors allow for subcellular localization within subcellular compartments and renal clearance. Moreover, in embodiments where drugs are attached to the nanosensors, treatment can be adjusted based on changes in the microenvironment.

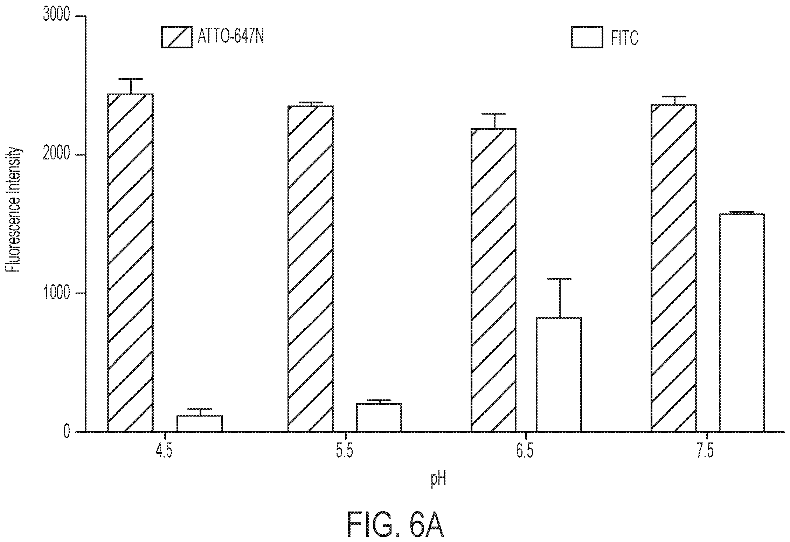

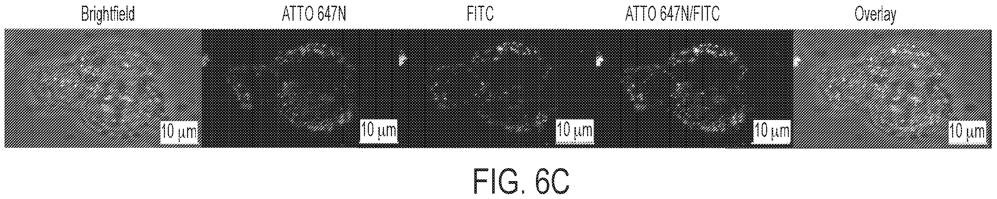

FIGS. 6A-C show plots (FIGS. 6A-6B) and images (FIG. 6C) depicting nanosensors for quantification of intracellular pH. Each of the nanosensors contained two dyes: a reference dye (ATTO-647N) located in the core of the silica-based nanoparticle and a sensor dye (FITC) located on the surface of the nanoparticle. The reference dye exhibits a relatively constant photo emission profile, whereas the sensor dye exhibits a different photon emission profile depending on pH conditions within the tissue. Therefore, this example describes quantitative measurement of environmental conditions within a tissue, and gleans more information from what was derived from earlier methods.

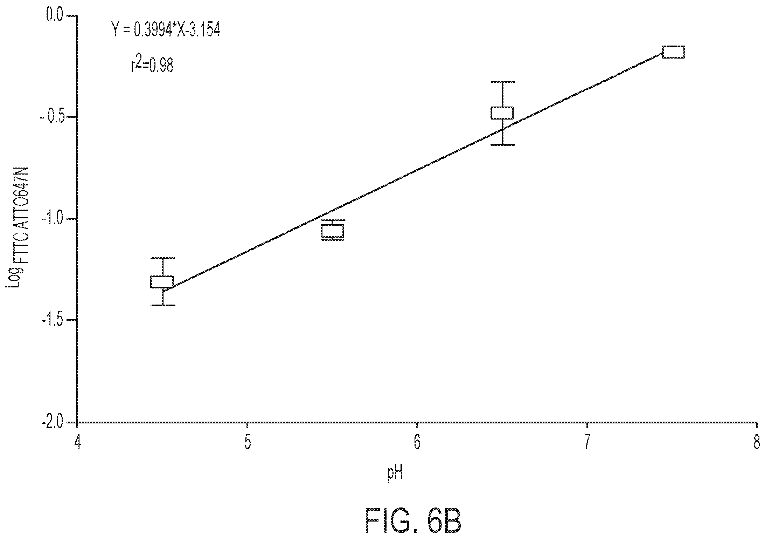

Ratiometric, pH sensing nanoparticles were administered to A431 (epidermoid carcinoma) cells at a concentration of 100 nM for 18 hrs prior to exposure to pH buffer solutions. FIG. 6A shows a raw signal intensity values of ATTO-647N dye (reference) and FITC dye (sensing) following exposure to pH buffer solutions collected using a microplate reader. FIG. 6B shows a standard curve of log-transformed FITC:ATTO-647N intensity ratios. FIG. 6C shows a representative confocal microscopy of A431 cells exposed to a pH 6.5 buffer solution (Scale bar = 10 $\mu$m).

## Claims

1. A method for imaging, surgical navigation, and/or cancer treatment planning, the method comprising:

   (a) administering to a tissue of a subject a composition comprising one or more nanoparticles,

   wherein the one or more nanoparticles comprise a silica-based core; a fluorescent compound within the core; a silica shell surrounding at least a portion of the core; and an organic polymer attached to the one or more nanoparticles, thereby coating the one or more nanoparticles,
   wherein each of the one or more nanoparticles operates as a nanosensor for one or more environmental conditions and/or analytes selected from the group consisting of reactive oxygen species (ROS), pH, pH perturbation, iron level, calcium, glutathione, leucine, glutamine, arginine, and other amino acid,

wherein each of the one or more nanoparticles has a diameter from about 1 nm to about 10 nm,

wherein each of the one or more or more nanoparticles comprises two or more photoluminescent dyes, the two or more photoluminescent dyes comprising at least one reference dye and at least one sensor dye, and

wherein the reference dye exhibits a relatively constant photon emission and the sensor dye exhibits different photon emissions depending on the one more environmental conditions; and

(b) detecting two or more signals emitted by the administered one or more nanoparticles after the one or more administered nanoparticles are localized within one or more subcellular compartments or structures, and/or across a blood brain barrier,

wherein at least one signal of the two or more signals is emitted by the reference dye and at least one signal of the two or more signals is emitted by the sensor dye,

wherein the at least one signal emitted by the sensor dye is indicative of one or more environmental conditions and/or analytes selected from the group consisting of reactive oxygen species (ROS), pH, pH perturbation, iron level, calcium, glutathione, leucine, glutamine, arginine, and other amino acid of the tissue, wherein the method comprises identifying the administered one or more nanoparticles in the tissue of the subject at a subcellular level, and

wherein identifying the administered one or more nanoparticles in the tissue of the subject at the subcellular level comprises counting individual nanoparticles.

2. The method of claim 1, wherein the reference dye and the sensor dye are chemically different dyes.

3. The method of any one of the preceding claims, wherein the reference dye and the sensor dye are separated in different compartments of the nanoparticle.

4. The method of any one of the preceding claims, wherein the administration is intravenous administration.

5. The method of any one of the preceding claims, the method further comprising:
   (c) determining, via a processor of a computing device, a quantitative map of one or more members selected from the group consisting of tissue perfusion, tissue viability, oxygen/pH status, deep tissue, and tumor volume, based on the detected signals, and displaying, via a graphical display, the map.

6. The method of claim 5, wherein the map is determined by a ratio of the signal emitted by the sensor dye normalized by the signal emitted by the reference dye.

7. The method of claim 1 further comprising
   (c) graphically rendering, via a processor of a computing device, based on the detected signal, a location of one or more nanoparticles localized within one or more cells of the tissue of the subject.

8. The method of any one of the preceding claims, comprising determining, based on the one or more nanoparticles, localized within one or more cells of the tissue of the subject, a dosing limit for drug delivery.

9. The method of any one of the preceding claims, wherein the nanoparticles have an average diameter from about 5 nm to about 7 nm.

10. The method of any one of the preceding claims, wherein the organic polymer is polyethylene glycol.

11. The method of any one of the preceding claims, wherein the nanoparticles comprise a member selected from the group consisting of C dots, C'dots, srC'dots, and iC'dots.

12. The method of any one of the preceding claims, wherein the nanoparticles comprise from 1 to 60 targeting moieties, wherein the targeting moieties bind to receptors on tumor cells.

13. The method of any one of the preceding claims, wherein the administered nanoparticles have a drug attached.

14. The method of claim 13, wherein the drug is attached via a linker moiety.

**Patentansprüche**

1. Ein Verfahren zur Bildgebung, chirurgischen Navigation und/oder Krebsbehandlungsplanung, wobei das Verfahren Folgendes umfasst:

(a) verabreichen einer Zusammensetzung, die ein oder mehrere Nanopartikel umfasst, an ein Gewebe eines Patienten,

wobei das eine oder die mehreren Nanopartikel einen Kern auf Siliziumdioxidbasis; eine fluoreszierende Verbindung innerhalb des Kerns; eine Siliziumdioxidhülle, die mindestens einen Teil des Kerns umgibt; und ein organisches Polymer, das an das eine oder die mehreren Nanopartikel gebunden ist, wodurch das eine oder die mehreren Nanopartikel beschichtet werden, umfasst

wobei jedes der einen oder mehreren Nanopartikel als Nanosensor für einen oder mehrere Umweltbedingungen und/oder Analyten arbeitet, die aus der Gruppe ausgewählt sind, die aus reaktiven Sauerstoffspezies (ROS), pH, pH-Störung, Eisengehalt, Kalzium, Glutathion, Leucin, Glutamin, Arginin und anderen Aminosäuren besteht,

wobei jedes der einen oder mehreren Nanopartikel einen Durchmesser von etwa 1 nm bis etwa 10 nm aufweist,

wobei jedes des einen oder der mehreren Nanopartikel zwei oder mehrere photolumineszente Farbstoffe umfasst, wobei die zwei oder mehreren photolumineszenten Farbstoffe mindestens einen Referenzfarbstoff und mindestens einen Sensorfarbstoff umfassen, und

wobei der Referenzfarbstoff eine relativ konstante Photonenemission aufweist und der Sensorfarbstoff unterschiedliche Photonenemissionen in Abhängigkeit von den einen oder anderen Umgebungsbedingungen aufweist; und

(b) erfassen von zwei oder mehr Signalen, die von dem verabreichten einen oder den mehreren Nanopartikeln emittiert werden, nachdem das eine oder die mehreren verabreichten Nanopartikel in einem oder mehreren subzellulären Kompartimenten oder Strukturen und/oder über eine Blut-Hirn-Schranke lokalisiert sind,

wobei mindestens ein Signal der zwei oder mehr Signale von dem Referenzfarbstoff emittiert wird und mindestens ein Signal der zwei oder mehr Signale von dem Sensorfarbstoff emittiert wird, wobei das mindestens eine von dem Sensorfarbstoff emittierte Signal eine oder mehrere Umgebungsbedingungen und/oder Analyten anzeigt, die aus der Gruppe ausgewählt sind, die aus reaktiven Sauerstoffspezies (ROS), pH, pH-Störung, Eisenspiegel, Kalzium, Glutathion, Leucin, Glutamin, Arginin und anderen Aminosäuren des Gewebes besteht,

wobei das Verfahren das Identifizieren des verabreichten einen oder der mehreren Nanopartikel im Gewebe des Subjekts auf subzellulärer Ebene umfasst, und

wobei das Identifizieren des verabreichten einen oder der mehreren Nanopartikel im Gewebe des Subjekts auf der subzellulären Ebene das Zählen einzelner Nanopartikel umfasst.

2. Das Verfahren nach Anspruch 1, wobei der Referenzfarbstoff und der Sensorfarbstoff chemisch unterschiedliche Farbstoffe sind.

3. Das Verfahren nach einem der vorhergehenden Ansprüche, wobei der Referenzfarbstoff und der Sensorfarbstoff in verschiedenen Kompartimenten des Nanopartikels getrennt sind.

4. Das Verfahren nach einem der vorangehenden Ansprüche, wobei die Verabreichung intravenös erfolgt.

5. Das Verfahren nach einem der vorangehenden Ansprüche, wobei das Verfahren ferner Folgendes umfasst: (c) bestimmen einer quantitativen Karte eines oder mehrerer Elemente, die aus der Gruppe ausgewählt sind, die aus Gewebeperfusion, Gewebelebensfähigkeit, Sauerstoff/pH-Status, tiefem Gewebe und Tumorvolumen besteht, basierend auf den erfassten Signalen, über einen Prozessor eines Rechengeräts, und Anzeigen der Karte über eine grafische Anzeige.

6. Das Verfahren nach Anspruch 5, wobei die Karte durch ein Verhältnis des von dem Sensorfarbstoff emittierten Signals, normiert durch das von dem Referenzfarbstoff emittierte Signal, bestimmt wird.

7. Das Verfahren nach Anspruch 1 umfasst ferner Folgendes
(c) grafisches Darstellen eines Ortes eines oder mehrerer Nanopartikel, die in einer oder mehreren Zellen des Gewebes des Subjekts lokalisiert sind, über einen Prozessor eines Rechengeräts, basierend auf dem detektierten Signal.

8. Das Verfahren nach einem der vorhergehenden Ansprüche umfasst die Bestimmung einer Dosierungsgrenze für die Arzneimittelabgabe auf der Grundlage des einen oder der mehreren Nanopartikel, die in einer oder mehreren Zellen des Gewebes des Patienten lokalisiert sind.

**9.** Das Verfahren nach einem der vorangehenden Ansprüche, wobei die Nanopartikel einen durchschnittlichen Durchmesser von etwa 5 nm bis etwa 7 nm aufweisen.

**10.** Das Verfahren nach einem der vorangehenden Ansprüche, wobei das organische Polymer Polyethylenglykol ist.

**11.** Das Verfahren nach einem der vorangehenden Ansprüche, wobei die Nanopartikel ein Element umfassen, das aus der Gruppe ausgewählt ist, die aus C-Punkten, C'-Punkten, srC'-Punkten und iC'-Punkten besteht.

**12.** Das Verfahren nach einem der vorhergehenden Ansprüche, wobei die Nanopartikel 1 bis 60 Targeting-Einheiten umfassen, wobei die Targeting-Einheiten an Rezeptoren auf Tumorzellen binden.

**13.** Das Verfahren nach einem der vorhergehenden Ansprüche, wobei an die verabreichten Nanopartikel ein Arzneimittel gebunden ist.

**14.** Das Verfahren nach Anspruch 13, wobei der Wirkstoff über einen Linker-Bruchteil gebunden ist.

**Revendications**

**1.** Procédé d'imagerie, de navigation chirurgicale et/ou de planification de traitement du cancer, le procédé comprenant :

(a) l'administration, à un tissu d'un sujet, d'une composition comprenant une ou plusieurs nanoparticules,

dans lequel les une ou plusieurs nanoparticules comprennent un noyau à base de silice ; un composé fluorescent à l'intérieur du noyau ; une coque de silice entourant au moins une partie du noyau ; et un polymère organique attaché aux une ou plusieurs nanoparticules, recouvrant ainsi les une ou plusieurs nanoparticules,
dans lequel chacune des une ou plusieurs nanoparticules fonctionne comme un nanocapteur pour une ou plusieurs conditions environnementales et/ou analytes sélectionnés dans le groupe constitué par les espèces réactives de l'oxygène (ROS), le pH, la perturbation du pH, le taux de fer, le calcium, le glutathion, la leucine, la glutamine, l'arginine et d'autres acides aminés,
dans lequel chacune des une ou plusieurs nanoparticules a un diamètre d'environ 1 nm à environ 10 nm,

dans lequel chacune des une ou plusieurs ou plusieurs nanoparticules comprend deux colorants photoluminescents ou plus, les deux colorants photoluminescents ou plus comprenant au moins un colorant de référence et au moins un colorant de capteur, et
dans lequel le colorant de référence présente une émission de photons relativement constante et le colorant de capteur présente des émissions de photons différentes en fonction des une plusieurs conditions environnementales ; et

(b) la détection de deux signaux ou plus émis par les une ou plusieurs nanoparticules administrées après que les une ou plusieurs nanoparticules administrées sont localisées à l'intérieur d'un ou plusieurs compartiments ou structures subcellulaires, et/ou à travers une barrière hémato-encéphalique,

dans lequel au moins un signal parmi les deux signaux ou plus est émis par le colorant de référence et au moins un signal parmi les deux signaux ou plus est émis par le colorant de capteur,
dans lequel l'au moins un signal émis par le colorant de capteur est indicatif d'une ou plusieurs conditions environnementales et/ou d'analytes sélectionnés dans le groupe constitué par les espèces réactives de l'oxygène (ROS), le pH, la perturbation du pH, le taux de fer, le calcium, le glutathion, la leucine, la glutamine, l'arginine et d'autres acides aminés du tissu,
dans lequel le procédé comprend l'identification des une ou plusieurs nanoparticules administrées dans le tissu du sujet à un niveau subcellulaire, et
dans lequel l'identification des une ou plusieurs nanoparticules administrées dans le tissu du sujet au niveau subcellulaire comprend le comptage de nanoparticules individuelles.

**2.** Procédé selon la revendication 1, dans lequel le colorant de référence et le colorant de capteur sont des colorants chimiquement différents.

**3.** Procédé selon l'une quelconque des revendications précédentes, dans lequel le colorant de référence et le colorant de capteur sont séparés dans différents compartiments de la nanoparticule.

**4.** Procédé selon l'une quelconque des revendications précédentes, dans lequel l'administration est une administration intraveineuse.

**5.** Procédé selon l'une quelconque des revendications précédentes, le procédé comprenant en outre :
(c) la détermination, via un processeur d'un dispositif informatique, d'une carte quantitative d'un ou de plusieurs éléments sélectionnés dans le groupe constitué par la perfusion tissulaire, la viabilité tissulaire, l'état d'oxygène/pH, le tissu profond et le volume tumoral, sur la base des signaux détectés, et l'affichage, via un affichage graphique, de la carte.

**6.** Procédé selon la revendication 5, dans lequel la carte est déterminée par un rapport du signal émis par le colorant de capteur normalisé par le signal émis par le colorant de référence.

**7.** Procédé selon la revendication 1 comprenant en outre
(c) la restitution graphique, via un processeur d'un dispositif informatique, sur la base du signal détecté, d'un emplacement d'une ou plusieurs nanoparticules localisées à l'intérieur d'une ou plusieurs cellules du tissu du sujet.

**8.** Procédé selon l'une quelconque des revendications précédentes, comprenant la détermination, sur la base des une ou plusieurs nanoparticules, localisées à l'intérieur d'une ou plusieurs cellules du tissu du sujet, d'une limite de dosage pour l'administration de médicament.

**9.** Procédé selon l'une quelconque des revendications précédentes, dans lequel les nanoparticules ont un diamètre moyen d'environ 5 nm à environ 7 nm.

**10.** Procédé selon l'une quelconque des revendications précédentes, dans lequel le polymère organique est le polyéthylène glycol.

**11.** Procédé selon l'une quelconque des revendications précédentes, dans lequel les nanoparticules comprennent un élément choisi dans le groupe constitué par les points C, les points C', les points srC' et les points iC'.

**12.** Procédé selon l'une quelconque des revendications précédentes, dans lequel les nanoparticules comprennent de 1 à 60 fractions de ciblage, dans lequel les fractions de ciblage se lient à des récepteurs sur des cellules tumorales.

**13.** Procédé selon l'une quelconque des revendications précédentes, dans lequel les nanoparticules administrées ont un médicament attaché.

**14.** Procédé selon la revendication 13, dans lequel le médicament est attaché via une fraction de liaison.

FIG. 1

FIG. 2

FIGS. 3A-B

FIGS. 4A-4B

FIG. 5A

FIG. 5B

FIG. 5C

FIG. 5D

FIG. 6A

FIG. 6B

FIG. 6C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62524441
- US 62590507
- US 8084001 B1, Burns
- WO 2018213851 A1
- US 1826980 W
- US 1833098 W
- US 8298677 B2
- US 20130039848 A1
- US 20140248210 A1
- US 20150343091 A1
- US 1730056 W, Bradbury
- US 20150182118 A1, Bradbury
- US 2016034351 W, Bradbury

### Non-patent literature cited in the description

- Stedman's Medical Dictionary. Williams & Wilkins, 1990